# EUROPEAN PATENT SPECIFICATION

(11) **EP 2 141 231 B1**
(45) Date of publication and mention of the grant of the patent: **12.03.2014**
(21) Application number: 08722734.4
(22) Date of filing: 24.03.2008
(51) Int. Cl.: C12N 15/09, A61K 31/7088, A61K 38/00, A61K 48/00, A61P 35/00, C12Q 1/02, C12Q 1/68, G01N 33/53

(54) **METHOD FOR INHIBITION OF SIGNALING THROUGH ErbB2, SIGNALING INHIBITOR FOR USE IN THE METHOD, AND USE OF THE SIGNALING INHIBITOR**
VERFAHREN ZUR HEMMUNG DER SIGNALGEBUNG ÜBER ERBB2, SIGNALGEBUNGSINHIBITOR ZUR VERWENDUNG IN DEM VERFAHREN UND VERWENDUNG DES SIGNALGEBUNGSINHIBITORS
PROCÉDÉ D'INHIBITION DE SIGNALISATION PAR L'INTERMÉDIAIRE D'ErbB2, INHIBITEUR DE SIGNALISATION DESTINÉ À ÊTRE UTILISÉ DANS CE PROCÉDÉ, ET UTILISATION DE L'INHIBITEUR DE SIGNALISATION

(30) Priority: 23.03.2007 WO PCT/JP2007/056100
(43) Date of publication of application: 06.01.2010
(73) Proprietor: The University of Tokyo, Bunkyo-Ku Tokyo 113-8654 (JP); TOKYO MEDICAL UNIVERSITY, Shinjyuku-ku Tokyo 160-8402 (JP); National University Corporation Tokyo Medical and Dental University, Bunkyo-ku Tokyo 113-8510 (JP)
(72) Inventor: GOTOH, Noriko, Tokyo 113-8654 (JP); KURODA, Masahiko, Tokyo 160-8402 (JP); TSUCHIDA, Nobuo, Tokyo 113-8510 (JP); WATANABE, Makoto, Tokyo 113-8654 (JP)
(74) Representative: Woods, Geoffrey Corlett
(86) International application number: PCT/JP2008/055468
(87) International publication number: WO 2008/123202

(56) References cited:
- WO-A1-2005/084691
- US-A1- 2006 019 296
- US-B1- 6 310 181
- MANUVAKHOVA M ET AL: "Expression of the SNT-1/FRS2 phosphotyrosine binding domain inhibits activation of MAP kinase and PI3-kinase pathways and antiestrogen resistant growth induced by FGF-1 in human breast carcinoma cells." ONCOGENE 28 SEP 2006 LNKD- PUBMED:16682955, vol. 25, no. 44, 28 September 2006 (2006-09-28), pages 6003-6014, XP002602377 ISSN: 0950-9232
- IEJIMA D ET AL: "FRS2beta, a potential prognostic gene for non-small cell lung cancer, encodes a feedback inhibitor of EGF receptor family members by ERK binding." ONCOGENE 27 MAY 2010 LNKD- PUBMED:20228838, vol. 29, no. 21, 27 May 2010 (2010-05-27), pages 3087-3099, XP002602378 ISSN: 1476-5594
- HUANG L. ET AL.: 'SNT-2 interacts with ERK2 and negatively regulates ERK2 signaling in response to EGF stimulation' BIOCHEM. BIOPHYS. RES. COMMUN. vol. 324, no. 3, 2004, pages 1011 - 1017, XP004599280
- OU R. ET AL.: 'EGF Yudo shita Saibo Transformation o Yokusei suru FRS2beta/SNT-2 no Yakuwari (NEGATIVE REGULATION OF EGF-INDUCED CELL TRANSFORMATION BY SNT-2)' DAI 64 KAI NIHON GAN GAKKAI GAKUJUTSU SOKAI KIJI, THE JAPANESE CANCER ASSOCIATION vol. 64TH, 2005, page 374 + ABSTR. NO. PP2-850, XP003018324
- HUANG L. ET AL.: 'Unique role of SNT-2/FRS2beta/FRS3 docking/adaptor protein for negative regulation in EGF receptor tyrosine kinase signaling pathways' ONCOGENE vol. 25, no. 49, 2006, pages 6457 - 6466, XP003018325
- OU R. ET AL.: 'FRS2beta/SNT-2 ni yoru EGF Juyotai Family Signal Dentatsu no Yokusei Seigyo (Unique role of FRS2beta/SNT-2 docking/adaptor protein for negative regulation of EGF receptor tyrosine kinase signaling pathways)' DAI 65 KAI NIHON GAN GAKKAI GAKUJUTSU SOKAI KIJI, THE JAPANESE CANCER ASSOCIATION vol. 65TH, 2006, pages 39 - 40 + ABSTR. NO. O-045, XP003018327

### SEQUENCE LISTING

<110> THE UNIVERSITY OF TOKYO TOKYO MEDICAL UNIVERSITY NATIONAL UNIVERSITY CORPORATION TOKYO MEDICAL AND DENTAL UNIVERSITY
<120> Method for inhibition of Er bB2 signaling, signaling inhibit or for use
   therin and use thereof
<130> TF08017-01
<150> PCT/ JP2007/ 056100
   <151> 2007-03-23
<160> 6
<170> PatentIn version 3.1
<210> 1
   <211> 492
   <212> PRT
   <213> Homo sapiens
<400> 1
   Met Gly Ser Cys Cys Ser Cys Leu Asn Arg Asp Ser Val Pro Asp Asn
   1               5                   10                  15
   His Pro Thr Lys Phe Lys Val Thr Asn Val Asp Asp Glu Gly Val Glu
               20                  25                  30
   Leu Gly Ser Gly Val Met Glu Leu Thr Gln Ser Glu Leu Val Leu His
           35                  40                  45
   Leu His Arg Arg Glu Ala Val Arg Trp Pro Tyr Leu Cys Leu Arg Arg
       50                  55                  60
   Tyr Gly Tyr Asp Ser Asn Leu Phe Ser Phe Glu Ser Gly Arg Arg Cys
   65                  70                  75                  80
   Gln Thr Gly Gln Gly Ile Phe Ala Phe Lys Cys Ser Arg Ala Glu Glu
                   85                  90                  95
   Ile Phe Asn Leu Leu Gln Asp Leu Met Gln Cys Asn Ser Ile Asn Val
               100                 105                 110
   Met Glu Glu Pro Val Ile Ile Thr Arg Asn Ser His Pro Ala Glu Leu
           115                 120                 125
   Asp Leu Pro Arg Ala Pro Gln Pro Pro Asn Ala Leu Gly Tyr Thr Val
       130                 135                 140
   Ser Ser Phe Ser Asn Gly Cys Pro Gly Glu Gly Pro Arg Phe Ser Ala
   145                 150                 155                 160
   Pro Arg Arg Leu Ser Thr Ser Ser Leu Arg His Pro Ser Leu Gly Glu
                   165                 170                 175
   Glu Ser Thr His Ala Leu Ile Ala Pro Asp Glu Gln Ser His Thr Tyr
   180 185 190
   Val Asn Thr Pro Ala Ser Glu Asp Asp His Arg Arg Gly Arg His Cys
   195 200 205
   Leu Gln Pro Leu Pro Glu Gly Gln Ala Pro Phe Leu Pro Gln Ala Arg
   210 215 220
   Gly Pro Asp Gln Arg Asp Pro Gln Val Phe Leu Gln Pro Gly Gln Val
   225 230 235 240
   Lys Phe Val Leu Gly Pro Thr Pro Ala Arg Arg His Met Val Lys Cys
   245 250 255
   Gln Gly Leu Cys Pro Ser Leu His Asp Pro Pro His His Asn Asn Asn
   260 265 270
   Asn Glu Ala Pro Ser Glu Cys Pro Ala Gln Pro Lys Cys Thr Tyr Glu
   275 280 285
   Asn Val Thr Gly Gly Leu Trp Arg Gly Ala Gly Trp Arg Leu Ser Pro
   290 295 300
   Glu Glu Pro Gly Trp Asn Gly Leu Ala His Arg Arg Ala Ala Leu Leu
   305 310 315 320
   His Tyr Glu Asn Leu Pro Pro Leu Pro Pro Val Trp Glu Ser Gln Ala
   325 330 335
   Gln Gln Leu Gly Gly Glu Ala Gly Asp Asp Gly Asp Ser Arg Asp Gly
   340 345 350
   Leu Thr Pro Ser Ser Asn Gly Phe Pro Asp Gly Glu Glu Asp Glu Thr
   355 360 365
   Pro Leu Gln Lys Pro Thr Ser Thr Arg Ala Ala Ile Arg Ser His Gly
   370 375 380
   Ser Phe Pro Val Pro Leu Thr Arg Arg Arg Gly Ser Pro Arg Val Phe
   385 390 395 400
   Asn Phe Asp Phe Arg Arg Pro Gly Pro Glu Pro Pro Arg Gln Leu Asn
   405 410 415
   Tyr Ile Gln Val Glu Leu Lys Gly Trp Gly Gly Asp Arg Pro Lys Gly
   420 425 430
   Pro Gln Asn Pro Ser Ser Pro Gln Ala Pro Met Pro Thr Thr His Pro
   435 440 445
   Ala Arg Ser Ser Asp Ser Tyr Ala Val Ile Asp Leu Lys Lys Thr Val
   450 455 460
   Ala Met Ser Asn Leu Gln Arg Ala Leu Pro Arg Asp Asp Gly Thr Ala
   465 470 475 480
   Arg Lys Thr Arg His Asn Ser Thr Asp Leu Pro Leu
   485 490
<210> 2
   <211> 1479
   <212> DNA
   <213> Homo sapiens
<400> 2
   atggggagct gctgcagctg cctgaacaga gacagcgttc cagacaacca ccccaccaag    60
   ttcaaggtga caaatgtgga tgatgagggg gtggagctgg gctctggggt gatggagctg   120
   acgcagagtg agctggtgct gcacctgcat cggcgtgagg ccgtccgctg gccttatctc   180
   tgcttgcggc gctatggcta cgactccaac ctcttctcct ttgagagtgg ccgccgatgt   240
   cagacaggcc agggaatatt tgcatttaag tgttcccggg ctgaggaaat cttcaacctc   300
   cttcaggatc tgatgcagtg caacagcatc aatgtgatgg aagagcctgt catcatcacc   360
   cgcaatagcc accccgctga gcttgacctc cctcgagccc cccagccacc caatgctcta   420
   ggctacactg tctccagctt ttccaatggc tgccctggag agggcccacg attctcagct   480
   ccccggcggc tctcgacaag cagcctgcgg cacccctcgc ttggggaaga gtccacccat   540
   gccctcattg ctcctgatga gcagtcccac acctatgtca acacaccggc cagtgaagat   600
   gaccaccgca ggggccgcca ctgcctgcag cccctgcctg agggtcaggc acccttcctc   660
   ccgcaggccc ggggacctga ccaacgggac ccacaggtgt tcttgcagcc aggccaggtg   720
   aagtttgtgt tgggcccgac ccctgctcgg cggcacatgg tgaagtgcca gggcctctgt   780
   cccagcctgc atgacccccc acaccacaat aataacaatg aggccccttc tgagtgtcca   840
   gcccagccca agtgcaccta cgagaacgtc accggggggc tgtggcgagg ggctggctgg    900
   agactgagcc cagaggagcc gggctggaat ggccttgccc accgccgggc cgccctgctg   960
   cactatgaga acctgccccc actgccccct gtgtgggaaa gccaagccca gcagctggga  1020
   ggggaggctg gggatgatgg ggactcgagg gatgggctca caccctcttc caatggcttc  1080
   cctgatggtg aggaggacga gaccccactg cagaagccca ccagcacccg ggccgccatc  1140
   cgcagccacg gcagctttcc tgtgccactg acccgccgcc gcggctcccc aagggtcttc  1200
   aactttgatt tccgccggcc ggggcccgag cccccaaggc agcttaacta catccaggtg  1260
   gagctaaagg gctggggtgg agaccgccct aaggggcccc agaacccctc gagcccccaa  1320
   gcccccatgc ccaccaccca ccctgcccga agctcagact cctacgccgt gattgacctc  1380
   aaaaagaccg tggccatgtc caacctgcag agagctctgc cccgagacga tggcaccgcc  1440
   aggaaaaccc ggcacaacag caccgacctg cctctgtag                          1479
<210> 3
   <211> 185
   <212> PRT
   <213> Homo sapiens
<400> 3
   Met Gly Ser Cys Cys Ser Cys Leu Asn Arg Asp Ser Val Pro Asp Asn
   1               5                   10                  15
   His Pro Thr Lys Phe Lys Val Thr Asn Val Asp Asp Glu Gly Val Glu
               20                  25                  30
   Leu Gly Ser Gly Val Met Glu Leu Thr Gln Ser Glu Leu Val Leu His
           35                  40                  45
   Leu His Arg Arg Glu Ala Val Arg Trp Pro Tyr Leu Cys Leu Arg Arg
       50                  55                  60
   Tyr Gly Tyr Asp Ser Asn Leu Phe Ser Phe Glu Ser Gly Arg Arg Cys
   65                  70                  75                  80
   Gln Thr Gly Gln Gly Ile Phe Ala Phe Lys Cys Ser Arg Ala Glu Glu
                   85                  90                  95
   Ile Phe Asn Leu Leu Gln Asp Leu Met Gln Cys Asn Ser Ile Asn Val
               100                 105                 110
   Met Glu Glu Pro Val Ile Ile Thr Arg Asn Ser His Pro Ala Glu Leu
           115                 120                 125
   Asp Leu Pro Arg Ala Pro Gln Pro Pro Asn Ala Leu Gly Tyr Thr Val
       130                 135                 140
   Ser Ser Phe Ser Asn Gly Cys Pro Gly Glu Gly Pro Arg Phe Ser Ala
   145                 150                 155                 160
   Pro Arg Arg Leu Ser Thr Ser Ser Leu Arg His Pro Ser Leu Gly Glu
                   165                 170                 175
   Glu Ser Thr His Ala Leu Ile Ala Pro
               180                 185
<210> 4
   <211> 555
   <212> DNA
   <213> Homo sapiens
<400> 4
   atggggagct gctgcagctg cctgaacaga gacagcgttc cagacaacca ccccaccaag     60
   ttcaaggtga caaatgtgga tgatgagggg gtggagctgg gctctggggt gatggagctg    120
   acgcagagtg agctggtgct gcacctgcat cggcgtgagg ccgtccgctg gccttatctc    180
   tgcttgcggc gctatggcta cgactccaac ctcttctcct ttgagagtgg ccgccgatgt    240
   cagacaggcc agggaatatt tgcatttaag tgttcccggg ctgaggaaat cttcaacctc 300
   cttcaggatc tgatgcagtg caacagcatc aatgtgatgg aagagcctgt catcatcacc 360
   cgcaatagcc accccgctga gcttgacctc cctcgagccc cccagccacc caatgctcta 420
   ggctacactg tctccagctt ttccaatggc tgccctggag agggcccacg attctcagct 480
   ccccggcggc tctcgacaag cagcctgcgg cacccctcgc ttggggaaga gtccacccat 540
   gccctcattg ctcct 555
<210> 5
   <211> 16
   <212> PRT
   <213> Homo sapiens
<400> 5
   Pro Gly Gln Val Lys Phe Val Leu Gly Pro Thr Pro Ala Arg Arg His
   1 5 10 15
<210> 6
   <211> 48
   <212> DNA
   <213> Homo sapiens
<400> 6
   ccaggccagg t gaagtttgt gttgggcccg acccctgct c ggcggcac 48

## Description

### TECHNICAL FIELD

The present invention relates to a method for inhibiting signaling, signaling inhibitor to be used therefor and use thereof.

### BACKGROUND ART

It has been widely known that receptor tyrosine kinases of signal transduction pathway play a role in the development of cancer. Among them, ErbB2 belonging to ErbB family and expressed in cell membrane are receptor-type proteins having tyrosine kinase activity, and their overexpression in various types of cancers have been reported. ErbB2 is sometimes referred to as HER2 or neu, and its over expression has been identified in breast cancer (10 - 30%), ovarian cancer (about 30%), bladder cancer (30 - 40%), and the like. As mentioned above, from the fact that the overexpression of ErbB2 plays a role in the development of cancer, antibodies against this receptor have been attempted to be used as a molecular-targeted anticancer drug. A specific example includes an antibody-ErbB2 antibody (common name: Trastuzumab, trade name: Herceptin), which targets an ErB2 protein and is used as a therapeutic drug for generally breast cancer. However, administration of the anticancer drug targeting ErbB2 is limited to the case of cancer which expresses the above-described receptors in large excess. Therefore, it has been desired to provide a new anticancer drug.

In addition, on the occasion of deciding on courses of treatment of a patient with breast cancer, for example, presence or absence of overexpression of ErbB2 (HER2) has to be determined in advance. The reason is that the patient to be applied with the above-described Herceptin is limited to the case in which the overexpression of ErbB2 is identified. For the method for testing the above-described overexpression of ErbB2, Hercep Test has been employed conventionally. This is a method of immunostaining of ErbB2 expressed on the surface of cell with monoclonal antibody, in which judgment is made by 4 levels of 0, 1+, 2+ and 3+, and the cases of 2+ and 3+ are evaluated as overexpression. As a detection method for this ErbB2, it is desirable to detect the activated ErbB2 through tyrosine phosphorylation. However, there is a problem that, when specific detection of phosphorylation site is carried out, other phosphorylated ErbB family is also detected. Therefore, there remains a problem that it is not clear whether the signaling pathway works actually by the phosphorylation of overexpressed ErbB2. Further, even when ErbB2 is expressed in large excess, if the signaling of ErbB2 does not function practically, the treatment by Harceptin will be inappropriate.

Non-patent Literature 1: Oncogene, 2006 Oct, 25(49), p.6457-66.
Non-patent Literature 2: Journal of Clinical Oncology, vol. 25, p587-595, 2007.

### SUMMARY OF THE INVENTION

And so, an object of the present invention is to provide a method for inhibiting activation of signaling pathway mediated by ErbB2 in a human cell and a signaling inhibitor to be used therefor. In addition, another object of the present invention is to provide an anticancer drug against cancer in which the signaling pathway mediated by ErbB2 in a human cell is involved, and a method for treating the same. Further, another obj ect of the present invention is to provide a method for determining whether the above-described signaling pathway mediated by the above ErbB2 is in function in a human cell.

To achieve the above-described objects, the anticancer drug of the present invention is:
- at least one polypeptide selected from the group consisting of polypeptides (A1), (B1), (A2), (B2) and (C1):
   (A1): a polypeptide comprising an amino acid sequence comprising the 1st - the 185th region in the amino acid sequence shown in SEQ ID NO: 1;
   (B1): a polypeptide comprising an amino acid sequence comprising the 232nd - the 252nd or the 237th - the 252nd region in the amino acid sequence shown in SEQ ID NO: 1;
   (A2): a polypeptide comprising an amino acid sequence in which 1-4 amino acid residues are deleted, replaced or added in the amino acid sequence of the 1st - the 185th region in the amino acid sequence shown in SEQ ID NO: 1, and having a function of binding to human ErbB2 equivalent to PTB domain of human FRS2β;
   (B2) : a polypeptide comprising an amino acid sequence in which 1-4 amino acid residues are deleted, replaced or added in the amino acid sequence of the 232nd - the 252nd or the 237th - the 252nd region in the amino acid sequence shown in SEQ ID NO: 1, and having a function of binding with human ERK2; and
   (C1) a polypeptide consisting of the amino acid sequence shown in SEQ ID NO: 1;
   for use in a method of treating cancer by administration of said anticancer drug to an individual in need thereof, wherein the cancer is characterised by overexpression of ErbB2 or is a cancer in which the signaling pathway mediated by ErbB2 is involved; or
- at least one nucleic acid encoding a respective polypeptide selected from the group consisting of polypeptides (A1), (B1), (A2) and (B2) as defined herein for use in a method of treating cancer by administration of said anticancer drug to an individual in need thereof, wherein the cancer is characterised by overexpression of ErbB2 or is a cancer in which the signaling pathway mediated by ErbB2 is involved.

The anticancer drug for use according to the invention may comprise at least one polypeptide selected from the group consisting of the following (A1), (A2), (B1) and (B2):
(A1) : a polypeptide consisting of an amino acid sequence comprising the 1st - the 185th region in the amino acid sequence shown in SEQ ID NO: 1;
(A2) : a polypeptide consisting of an amino acid sequence in the above-described (A1) in which one or several amino acid residues are deleted, replaced or added, and which has a function equivalent to PTB domain of human FRS2β;
(B1) : a polypeptide consisting of an amino acid sequence comprising the 232nd - the 252nd region or the 237th - the 252nd region in the amino acid sequence shown in SEQ ID NO: 1;
(B2) : a polypeptide consisting of an amino acid sequence of the above-described (B1) in which one or several amino acid residues are deleted, replaced or added, and which has a function of binding with human ERK2.

The anticancer drug may comprise at least one nucleic acid selected from the group consisting of the following (a1), (a2), (b1) and (b2), and expresses coded polypeptide thereof in the cell:
(a1) : nucleic acid consisting of a nucleotide sequence comprising the 1st - the 555th region of the nucleotide sequence shown in SEQ ID NO: 2;
(a2): nucleic acid consisting of the nucleotide sequence of the above-described (a1) in which one or several nucleotides are deleted, replaced or added, and which encode for a polypeptide having a function equivalent to PTB domain of human FRS2β;
(b1) : nucleic acid consisting of a nucleotide sequence comprising the 709th - the 756th region of the nucleotide sequence shown in SEQ ID NO: 2;
(b2): nucleic acid consisting of the nucleotide sequence of the above-described (b1) in which one or several nucleotides are deleted, replaced or added, and encode for a polypeptide having a function of binding with human ERK2.

The anticancer drug for use according to the invention may comprise at least one of the nucleic acids of (a1) and (a2) and at least one of the nucleic acids of (b1) and (b2). For example, it may be a chimeric nucleic acid.

The invention also provides the use of at least one polypeptide selected from the group consisting of the above-described (A1), (A2), (B1) and (B2) or at least one nucleic acid as defined herein encoding a respective said polypeptide for the preparation of a medicament for the treatment of cancer, wherein the cancer is characterized by overexpression of ErbB2 or is a cancer in which the signaling pathway mediated by ErbB2 is involved.

The anticancer drug of the present invention is an anticancer drug for humans.

The invention provides a substance selected from an antibody specific for human FRS2β, a probe specific for human FRS2β gene and a primer specific for human FRS2β gene, for use in a method of diagnosing the necessity of treatment of a human cancer cell with an anticancer drug, wherein when down-regulation of FRS2β signaling is detected, treatment with an ErbB2-targeted anti-cancer drug is necessary.

The *in vitro* method of determination of the present invention is a method for determining the presence of the down-regulation of a signaling pathway, mediated by ErbB2 in a human cell, which comprises a step of detecting of the down-regulation marker comprising at least one of human FRS2β and transcription product of FRS2β gene, wherein detection of FRS2β indicates that the ErbB2 signalling pathway is down-regulated.

The *in vitro* method of diagnosis of the present invention is a method of diagnosis for making judgment on the necessity of treatment of cancer with an anticancer drug, and comprises a step of detection of a marker comprising at least one of human FRS2β and transcription product of FRS2β gene, wherein when down-regulation of FRS2β signaling is detected, treatment with an ErbB2-targeted anti-cancer drug is necessary.

The marker may be detected using an antibody specific for human FRS2β, a probe specific for human FRS2β gene and a primer specific for human FRS2β gene.

The present inventors have found, as the results of intensive study, that the FRS2β down-regulates the signaling of ErbB2. And further, the present inventors have found that any of polypeptide (hereinafter, referred to "polypeptide of the present invention") having at least one of PTB domain (phosphorylated tyrosine binding domain) of FRS2β and a binding domain of FRS2β with ERK2 (extracellular signal regulation kinase 2) (hereinafter, referred to as "ERK2 binding domain") may cause the above-described down-regulation. It should be noted that the ERK2 binding domain of FRS2β is the one which has been identified by the present inventors.

As mentioned above, the above-described signaling pathway mediated by ErbB2 may be down-regulated by the FRY2β polypeptides described herein. Therefore, for example, by introducing the FRS2β polypeptide or the nucleic acid capable of expressing the FRS2β polypeptide into an objective human cell, the above-described signaling in the above-described cell can be suppressed. Further, from the fact that the signaling of ErbB2 is involved in development of cancer as described above, prevention of development of the cancer and also treatment of the cancer may be performed by way of inhibition of signaling according to the present invention. In addition, since, for example, malignant alteration of cell as well as proliferation and growth of cell can be suppressed by the above-described inhibition of signaling, it can also be said that the present invention is useful for prevention and treatment of cancer.

In addition, because of the fact that the signaling of ErbB2 is down-regulated by FRS2β, determination on whether the above-described signaling is working can also be performed by detecting FRS2β in the target cell. And, on the basis of the result of the determination, for example, judgment on the necessity of treatment with molecularly-targeted drug such as Harceptin which inhibits the function of ErbB2 can be made. As stated above, it can be said that the present invention is a quite useful technology, for example, in the fields of medical treatment and molecular cytology.

### BRIEF DESCRIPTION OF THE DRAWINGS

Fig. 1:

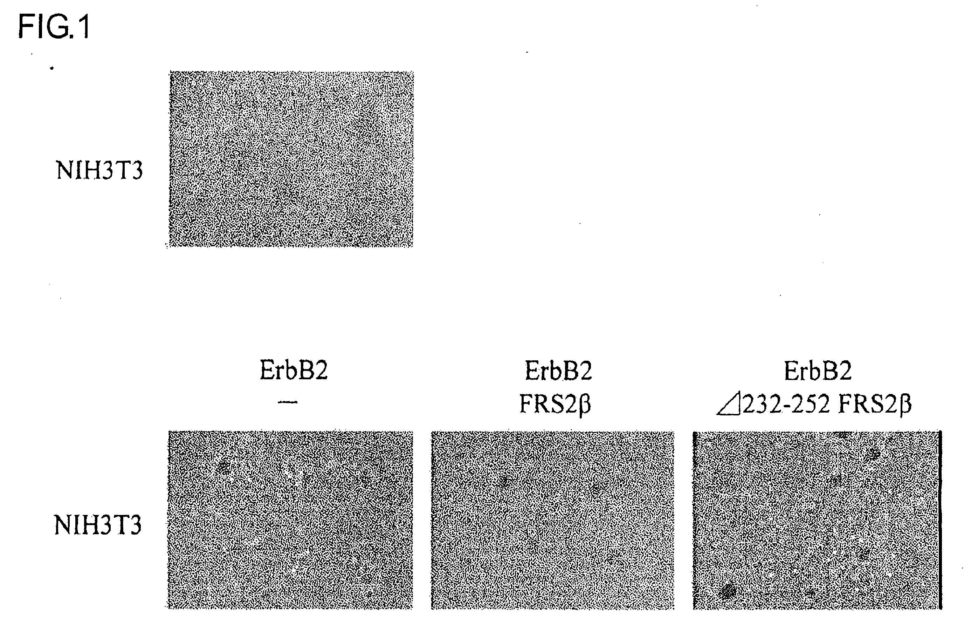

   Fig. 1 shows photographs of colony morphologies of NIH3T3 cells, in one Example of the present invention.
Fig. 2:

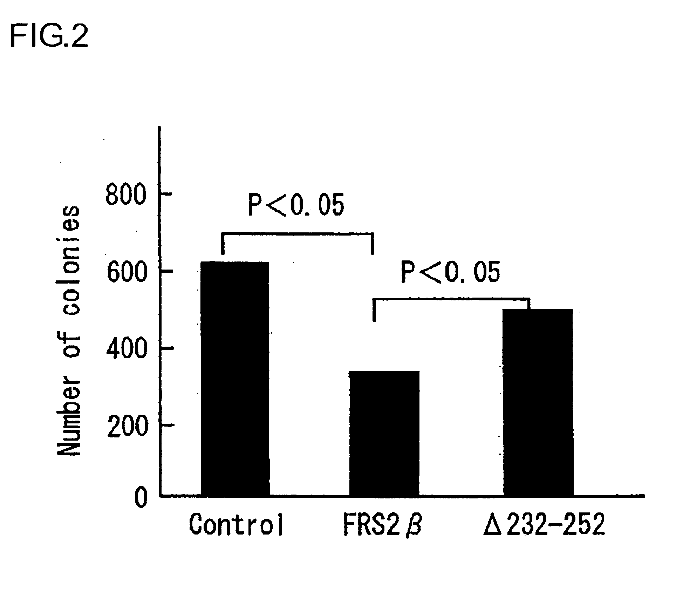

   Fig. 2 is a graphical presentation of the number of colonies of NIH3T3 cell, in the above-described Example.
Fig. 3:

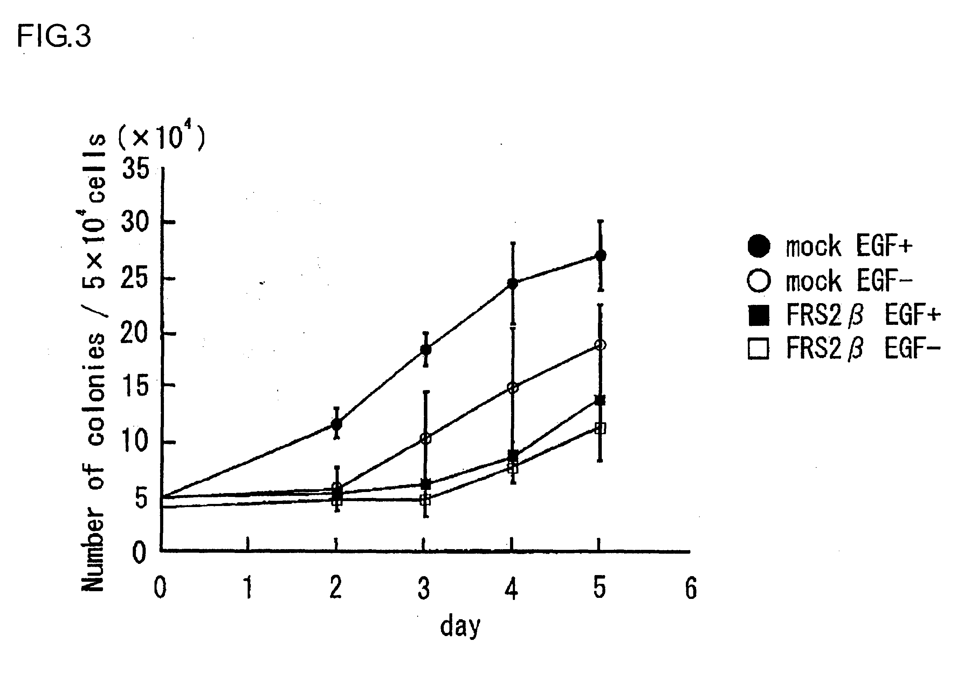

   Fig. 3 is a graphical presentation of the proliferation of the cells expressed FRS2β with time, in other Example of the present invention.
Fig. 4:

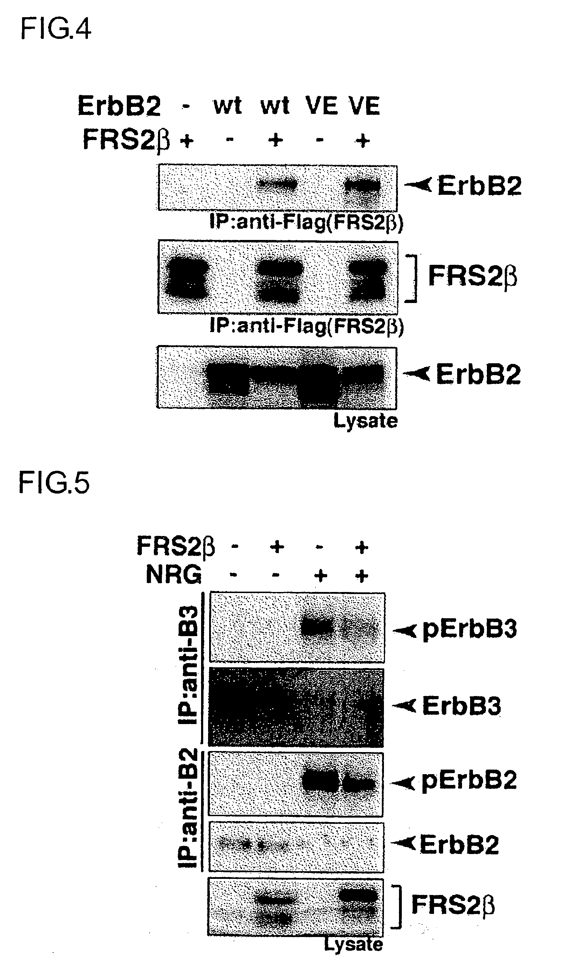

   Fig. 4 shows photographs representing the results of immunoblotting of HEK293T cells which express the ErbB2 and FRS2β, in yet another Example of the present invention.
Fig. 5:
   Fig. 5 shows photographs representing the results of immunoblotting of the MCF-7 cells, in yet another Example of the present invention.
Fig. 6:

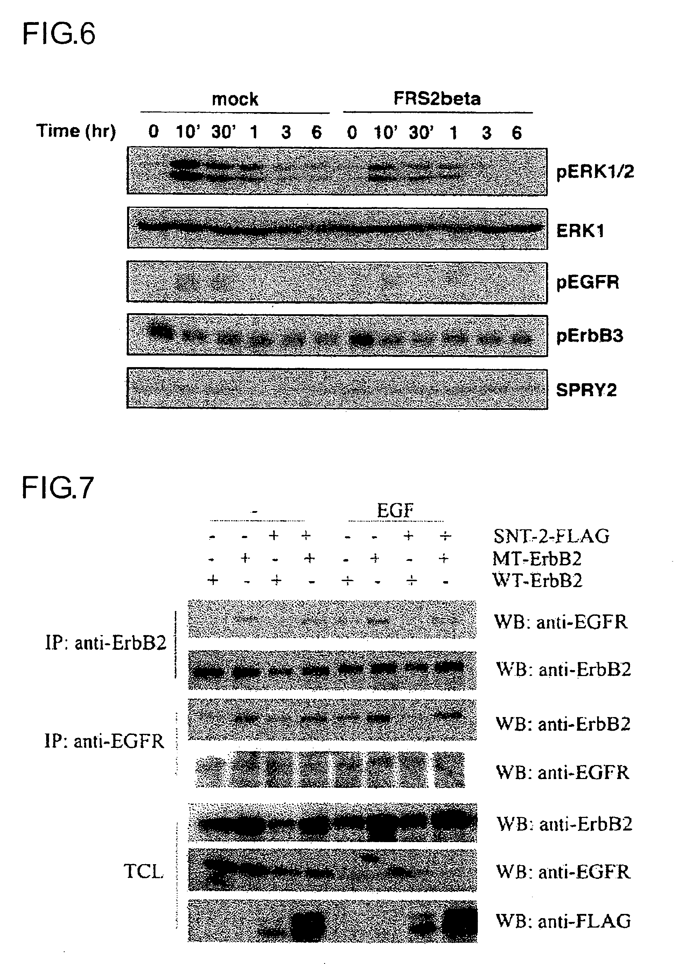

   Fig. 6 shows photographs representing the results of immunoblotting of the BT474 cells which express FRS2β, in yet another Example of the present invention.
Fig. 7:
   Fig. 7 shows photographs representing the results of immunoblotting of the HEK293T cells which express ErbB2 and FRS2β, in yet another Example of the present invention.
Fig. 8:

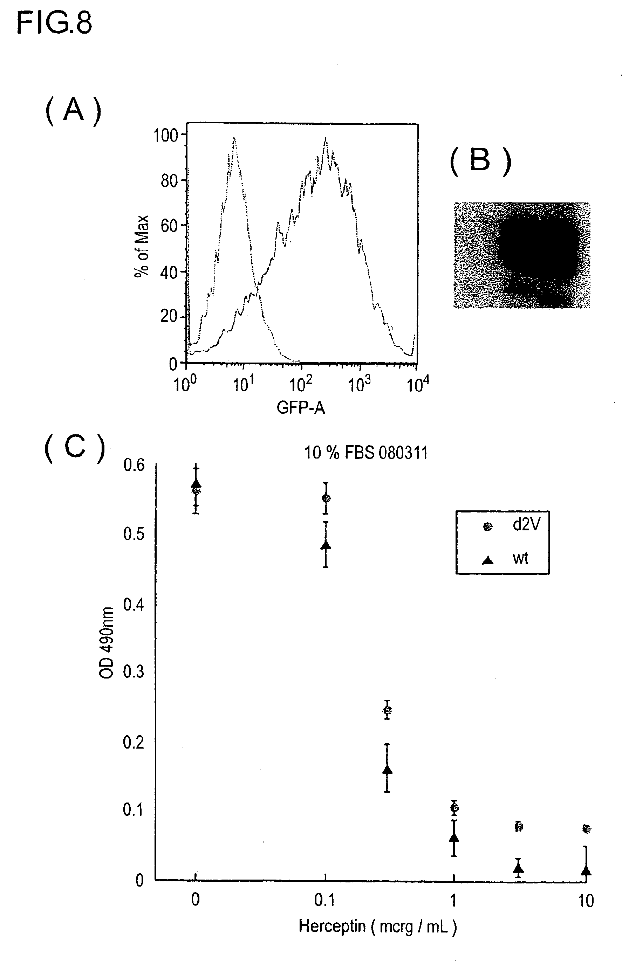

   In Fig. 8, (A) shows graphs representing the results of isolation of d2Venus expressing cells by FACS after transfection of the d2Venus expressing Lentivirus, in order to examine the multiplicity of infection by Lentivirus to human breast cancer cell line BTB474 which is overexpessing endogenously ErbB2, in yet another Example of the present invention; (B) shows a photograph of electrophoresis of lysates derived from cells expressing FRS2β and control cells expressing d2Venus; and (C) is a graphical presentation of the relationship between the concentration of Herceptin and absorbance of cultured cells.
Fig. 9:

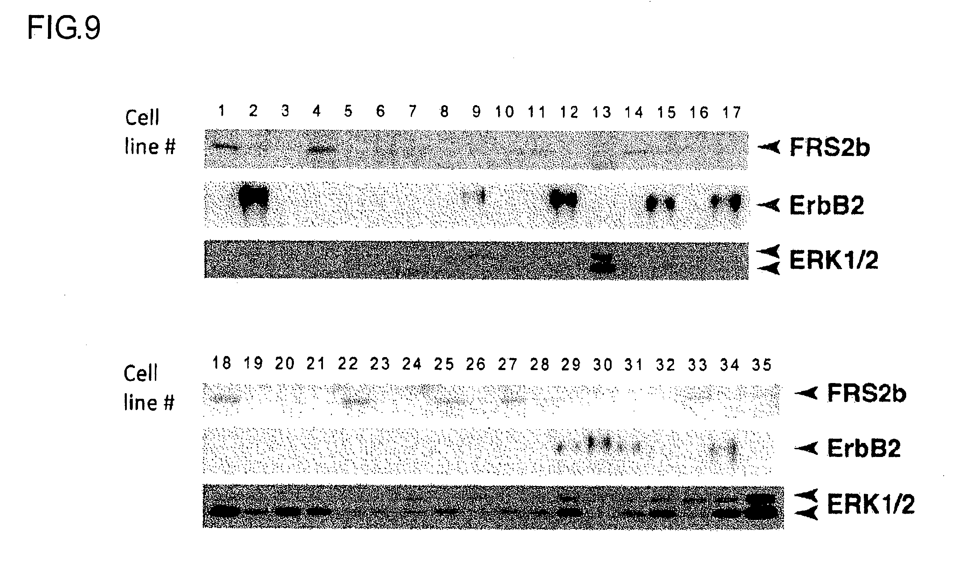

   Fig. 9 shows a photograph representing the results of immunoblotting of the breast cancer cells, in yet another Example of the present invention.
Fig. 10:

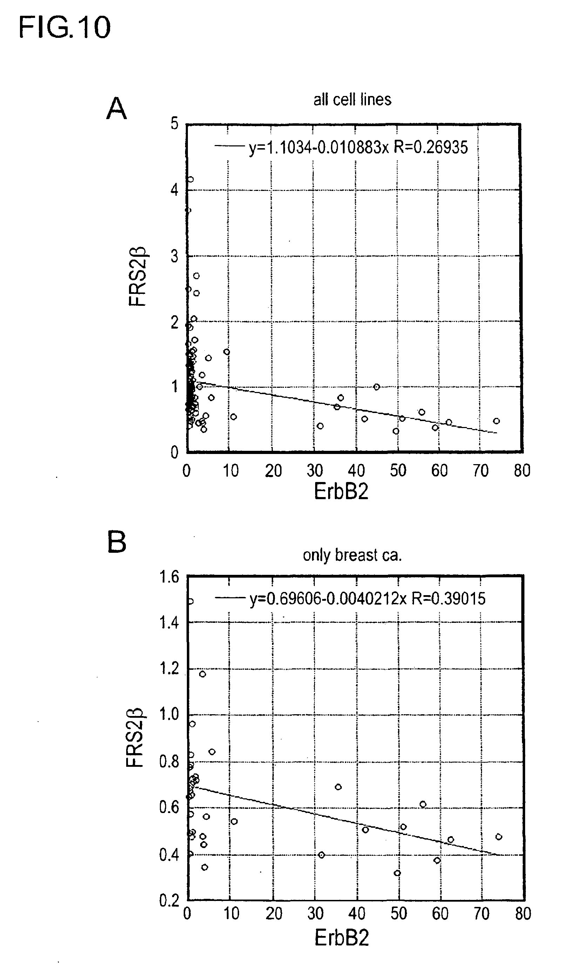

   In Fig. 10, (A) shows photograph representing the results of microarray analysis of the various cancer cells, in yet another Example of the present invention; (B) shows photograph representing the results of microarray analysis of the breast cancer cells.

### BEST MODE FOR CARRYING OUT THE INVENTION

### <Method for inhibiting signaling>

The method for inhibiting signaling of the present invention is, as previously mentioned, a method by which the activation of the signaling pathway is inhibited, wherein the above-described signaling pathway is a signaling pathway mediated by ErbB2 in a human cell, and the above-described activation of signaling pathway is inhibited by at least one polypeptide selected from the above-described group consisted of (A1), (A2), (B1) and (B2).
(A1): A polypeptide consisted of an amino acid sequence comprising the 1st - the 185th region in the amino acid sequence shown in SEQ ID NO: 1.
(A2) : In the amino acid sequence of the above-described (A1), the polypeptide is consisted of an amino acid sequence in which one or several amino acid residues are deleted, replaced or added, and which has a function equivalent to PTB domain of human FRS2β.
(B1): A polypeptide consisted of an amino acid sequence comprising the 232nd - the 252nd region or the 237th - the 252nd in the amino acid sequence shown in SEQ ID NO: 1.
(B2) : In the amino acid sequence of the above-described (B1), the polypeptide is consisted of an amino acid sequence in which one or several amino acid residues are deleted, replaced or added, and which has a function of binding with human ERK2.

The ErbB2 is, as mentioned previously, a receptor-type tyrosine kinase belonging to ErbB family that participates in signaling. The ErbB2 is a receptor having no specific corresponding ligand, different from the ErbB1 of the same family for EGF (epidermal growth factor) and the like as a ligand. In this ErbB2, homodimerization or heterodimerization with other receptor is induced by stimulation from outside, and thereby the kinase domain in the intracellular domain of ErbB2 is activated. ErbB2 is sometimes referred to as HER2 or neu.

Human FRS2β is sometimes referred to as SNT-2 or FRS3. FRS2β is a docking protein involved in signaling of FGFR (fibroblast cell growth factor receptor) or neutrophine receptor, and the following things have been known. FRS2β is coupled to the lipid of cell membrane through myristylation signal possessed on the N-terminal thereof. Ans, when the FGFR or neutrophin receptor is activated, FRS2β will bind with intracellular domain of the above-described receptor through PTB domain (phosphorylated tyrosine binding domain) of the FRS2β. The FRS2β bound with the above-described receptor will further receive phosphorylation of tyrosine residue thereof. When the FRS2β receives phosphorylation, various types of signaling molecules (e.g., Shp2, Grb2 and the like) bind to this tyrosine phosphorylation domain, and then these signaling molecules are activated. And, activation of these signaling molecules may give rise to, for example, activation of Ras-ERK pathway and the like. However, the FRS2β shows completely different function from that reported for signaling of the above-described FGFR in the signaling of ErbB2. And, there is no report on anything about this matter. The fact that, in the signaling of ErbB2, FRS2β does not give rise to activation of signaling but to down-regulation was found first by the present inventors as mentioned previously. And, the present inventors have further found that, according to at least one of PTB domain and a domain capable of binding with ERK2 (ERK2 binding domain) in the FRS2β, the above-described down-regulation can be realized. Specifically, down-regulation of signaling is taken place in such a way that, for example, autophosphorylation of ErbB2, and phosphorylation of downstream molecules are inhibited.

Amino acid sequence of human FRS2β, the entire length of human FRS2β gene and CDS sequence (including stop codon) encoding for human FRS2β and the like have been registered, for example, in NCBI Accession No. MN_006653. Amino acid sequence of human FRS2β and CDS sequence of human FRS2β gene are shown in SEQ ID NO: 1 and SEQ ID NO: 2, respectively. The PTB domain of human FRS2β is the 1st - the 185th region (SEQ ID NO: 3) in amino acid sequence of SEQ ID NO: 1, and coding sequence thereof is the 1st - the 555th region (SEQ ID NO: 4) in cDNA sequence of SEQ ID NO: 2. In addition, the ERK2 binding domain of human FRS2β is the 237th - the 252nd region (SEQ ID NO: 5) in amino acid sequence of SEQ ID NO: 1, and coding sequence thereof is the 709th - the 756th region (SEQ ID NO: 6) in cDNA sequence of SEQ ID NO: 2.

The polypeptide of the present invention may be a polypeptide which has at least one of the functions of a function of PTB domain and a function of binding with human ERK2.

First, the polypeptide having a function of PTB domain includes, for example, the polypeptides shown in the following (A1) - (A2).
(A1) : A polypeptide consisted of an amino acid sequence comprising the 1st - the 185th region in the amino acid sequence shown in SEQ ID NO: 1.
(A2) : In the amino acid sequence of the above-described (A1), the polypeptide is consisted of an amino acid sequence in which one or several amino acid residues are deleted, replaced or added, and which has a function equivalent to PTB domain of human FRS2β.

The "function equivalent to PTB domain" in the above-described (A2) includes, for example, the function of binding to human ErbB2. In the above-described (A2), the number of amino acid residue which can be deleted, replaced or added is not particularly limited, for example, as long as it is within a range of not losing the function of PTB domain of the human FRS2β. The number of amino acid residue acceptable for the above-described deletion and the rest is, for example, preferably 1 - 4 residues, more preferably 1 - 3 residues and further preferably 1 - 2 residues for 50 amino acid residues. In addition, the homology of amino acid sequence to the polypeptide of the above-described (A1) is, for example, 70% or higher.

The polypeptide of the above-described (A1) may be, for example, a polypeptide comprising PTB domain, as mentioned previously, or a polypeptide consisted of only PTB domain shown in the following (A3).
(A3): A polypeptide consisted of an amino acid sequence of the 1st - the 185th in the amino acid sequence (SEQ ID NO: 3) shown in SEQ ID NO: 1.

Next, the polypeptide having a function of ERK2 binding domain includes, for example, the polypeptides shown in the following (B1) - (B2).
(B1): A polypeptide consisted of an amino acid sequence comprising the 232nd - the 252nd or 237th - the 252nd region in the amino acid sequence shown in SEQ ID NO: 1.
(B2) : In the amino acid sequence of the above-described (B1), the polypeptide is consisted of an amino acid sequence in which one or several amino acid residues are deleted, replaced or added, and which has a function of binding with human ERK2.

In the above-described (B2), the number of amino acid residue which can be deleted, replaced or added is not particularly limited, for example, as long as it is within a range of not losing the function of human ERK2 binding domain. The number of amino acid residue acceptable for the above-described deletion and the rest is, for example, preferably 1 - 4 residues, more preferably 1 - 3 residues and further preferably 1 - 2 residues for 50 amino acid residues. In addition, homology of amino acid sequence to the polypeptide of the above-described (B1) is, for example, 70% or higher.

The above-described polypeptide having a function of ERK2 binding domain may be, for example, a polypeptide comprising ERK2 binding domain as mentioned previously, or a polypeptide consisted of only PTB domain shown in the following (B3).
(B3) : A polypeptide consisted of an amino acid sequence of the 232nd - the 252nd or 237th - the 252nd in the amino acid sequence (SEQ ID NO: 5) shown in SEQ ID NO: 1.

In addition, the above-described polypeptide having a function of ERK2 binding domain may be, for example, a polypeptide shown in the following (B4).
(B4): A polypeptide consisted of an amino acid sequence including at least one amino acid at 250th, 251st, 315th and 316th region of the sequence shown in SEQ ID NO: 1.

The amino acids at 250th, 251st, 315th and 316th region in SEQ ID NO: 1 are the binding sites with ERK2 in FRS2β, which have been determined by the present inventors. The above-described polypeptides include, for example, peptides including amino acids at 250th -251st and amino acids at 315th - 316th, and 250th - 316th region. In addition, in the present invention, it may be the amino acids at 250th, 251st, 315th and 316th region in the amino acid sequence shown in SEQ ID NO: 1, in place of a polypeptide having the above-described ERK2 binding function.

The polypeptide in the present invention may be either one of polypeptides of a polypeptide having a function of PTB domain as mentioned previously and a polypeptide having a function of ERK2 binding domain, or may be both polypeptides. In addition, the polypeptide in the present invention may be a polypeptide having both functions of PTB domain and ERK2 binding domain. The above-described polypeptide having functions of both domains includes, for example, polypeptides shown in the following (C1) - (C3). By the way, in the following (C3), the number of amino acid acceptable for deletion and the like is, for example, the same as mentioned previously.
(C1): Human FRS2β consisted of amino acid sequence shown in SEQ ID NO: 1.
(C2): A fused polypeptide comprising at least one of polynucleotide of the above-described (A1) and (A2) and at least one of polypeptide of the above-described (B1) and (B2).
(C3) : In the amino acid sequence of the above (C1) and (C2), a polypeptide consisted of an amino acid sequence in which one or several amino acid residues are deleted, replaced or added, and which has functions of both PTB domain and human ERK2 binding domain.

As mentioned previously, in the signaling pathway mediated through ErbB2 in a human cell, down-regulation occurs by the presence of at least one of polypeptide having a function of PTB domain and a polypeptide having a function of ERK2 binding domain. Therefore, in the present invention, a polypeptide having at least one of the domains may be present in the target human cell. For this reason, in the present invention, for example, the nucleic acid which encode for the above-described polynucleotide is introduced into the target cell, and thereby, the above-described polypeptide may be expressed in the cell, or the polypeptide may be administrated into the target cell. And so, as the first embodiment, introduction of signaling inhibitor comprising nucleic acid; and as the second embodiment, introduction of signaling inhibitor comprising polypeptide; will be each described. It should be noted that these are only for illustration, and the present invention is not limited thereto.

### First embodiment (administration of nucleic acid)

The method for inhibiting signaling of the present invention comprises a step of administration of a signaling inhibitor comprising nucleic acid into human cell, wherein the above-described nucleic acid encodes for the above-described polypeptide and expresses the above-described polypeptide in the cell. In this regard, hereinafter, the signaling inhibitor comprising the above-described nucleic acid is sometimes referred to as "the first signaling inhibitor" in the present invention.

The above-described nucleic acid may be the nucleic acid which encodes for the above-described polypeptide. Type of the nucleic acid is not limited, and it may be, for example, either one of DNA, RNA, mRNA, siRNA, and the like. In addition, the above-described nucleic acid may be, for example, naturally-occurring nucleic acid, or synthesized nucleic acid by means of genetic engineering.

First, the nucleic acid which encodes for a polypeptide having a function of PTB domain includes, for example, the nucleic acid shown in the following (a1) and (a2).
(a1): Nucleic acid consisted of a nucleotide sequence comprising the 1st - the 555th region of the nucleotide sequence shown in SEQ ID NO: 2;
(a2): Nucleic acid consisted of the nucleotide sequence of the above-described (a1) in which one or several nucleotides are deleted, replaced or added, and encoding for a polypeptide having a function equivalent to PTB domain of human FRS2β.

In the above-described (a2), the number of polynucleotide which can be deleted, replaced or added is not particularly limited, for example, as long as it is within a range of not losing the function of PTB domain encoded by the nucleic acid. The above-described number of nucleotide is not particularly limited, but is, for example, preferably 1 - 6 nucleotides, more preferably 1 - 5 nucleotides, further preferably 1 - 4 nucleotides, and particularly preferably 1 - 3 nucleotides for 50 nucleotides.

The nucleic acid shown in the above (a2) may be, as long as it is within a range of not losing the function of PTB domain, for example, the nucleic acid which is capable of hybridizing with the nucleic acid described in the above (a1) under stringent condition, or the nucleic acid which has homology to the nucleic acid described in the above (a1) in 90% or higher. The stringent condition for hybridization includes, for example, the standard condition in the field of relevant art; for example, temperature condition is +/-5°C of Tm value of the nucleic acid of the above-described (a1), preferably +/-2°C and more preferably +/-1°C. Specific example of the condition includes, the hybridization in 5 x SSC solution, 10 x Denhardt solution, 100 µg/ml salmon sperm DNA and 1% SDS, at 65°C, and washing (twice) in 0.2 x SSC and 1% SDS, at 65°C for 10 minutes. In addition, homology is, for example, 90% or higher, preferably 95% or higher, more preferably 97.5% or higher.

The nucleic acid of the above-described (a1) may be, for example, the nucleic acid comprising coding sequence for the PTB domain as mentioned previously, or the nucleic acid consisted of only a coding sequence for PTB domain shown in the following (a3).
(a3) : Nucleic acid consisted of a nucleotide sequence of the 1st - the 555th (SEQ ID NO: 4) in the nucleotide sequence shown in SEQ ID NO: 2.

In addition, nucleic acid which encodes for polypeptide having a function of the above-described PTB domain may be, for example, a nucleic acid shown in the following (a4) or (a5).
(a4): Nucleic acid which encodes for amino acid sequence including 1st - 185th region in the amino acid sequence shown in SEQ ID NO: 1.
(a5): Nucleic acid in which one or several nucleotides are deleted, replaced or added, and encode for a polypeptide having a function equivalent to PTB domain of human FRS2β, in the nucleotide sequence of the above-described (a4).

Next, the nucleic acid which encodes for a polypeptide having the function of ERK2 binding domain includes, for example, the nucleic acids shown in the following (b1) - (b2) .
(b1): Nucleic acid consisted of a nucleotide sequence comprising the 709th - the 756th region of the nucleotide sequence shown in SEQ ID NO: 2;
(b2): Nucleic acid consisted of the nucleotide sequence of the above-described (b1) in which one or several nucleotides are deleted, replaced or added, and encoding for a polypeptide having a function of binding with human ERK2.

In the above described (b2), the number of nucleotide which can be deleted, replaced or added is not particularly limited, for example, as long as the polynucleotide encoded by the nucleic acid is within a range of not losing the function of ERK2 binding domain. The above-described number of nucleotide is, for example, the same as mentioned previously.

The nucleic acid shown in the above (b2) may be, for example, as long as it is within a range of not losing the function of ERK2 binding domain, the nucleic acid which is capable of hybridizing with the nucleic acid described in the above (b1) under stringent condition, or the nucleic acid which has homology to the nucleic acid described in the above (b1) in 90% or higher. The above-described stringent condition is the same as described above, and the preferable range of the above-described homology is also the same as described above.

The nucleic acid which encodes the polypeptide having a function of the above-described ERK2 binding domain may be, for example, the nucleic acid comprising coding sequence for the ERK2 binding domain as mentioned previously, or the nucleic acid consisted of only a coding sequence for the ERK2 binding domain shown in the following (b3).
(b3): The nucleic acid consisted of a nucleotide sequence of the 709th - the 756th (SEQ ID NO: 6) in the nucleotide sequence shown in SEQ ID NO: 2.

In addition, nucleic acid which encodes a polypeptide having the function of the above-described ERK2 binding domain may be, for example, at least one polypeptide selected from the group consisted of the following (b4) - ( b6).
(b4): Nucleic acid which encodes for an amino acid sequence comprising the 232nd - the 252nd or the 237th - the 252nd region in the amino acid sequence shown in SEQ ID NO: 2.
(b5) : Nucleic acid which encodes for the amino acid sequence including at least one of the amino acids at the 250th, 251st, 315th and 316th region in the amino acid sequence shown in SEQ ID NO: 1, and nucleic acid which encodes for a polypeptide having a function of binding with human ERK2.
(b6) : In the nucleotide sequence of the above-described (b4) or (b5), nucleic acid consisted of nucleotide sequence in which one or several nucleotides are deleted, replaced or added, and encodes for a polypeptide having a function of binding with human ERK2.

The nucleic acid of above-described (b5) includes, for example, nucleic acid which encodes for peptides comprising amino acids at the 250th- the 251st region, peptides comprising amino acids at the 315th - the 316th region and peptides comprising amino acids at the 250th - the 316th region.

The above-described nucleic acid, for example, may be either one of nucleic acid which encode for a polypeptide having a function of PTB domain as mentioned previously or nucleic acid which encode for a polypeptide having a function of ERK2 binding domain, or may be both nucleic acids. In addition, in the present invention, the above-described nucleic acid may be nucleic acid which encode for a polypeptide (including protein) having functions of both PTB domain and ERK2 binding domain. The latter polypeptide includes, for example, polypeptides shown in the following (c1) - (c3). By the way, in the following (c3), the number of nucleotide corresponding to deletion and the like is, for example, the same as mentioned previously. In addition, it may be the entire length or a partial sequence of human Fry2β gene.
(c1): Human FRS2β gene consisted of a nucleotide sequence shown in SEQ ID NO: 2.
(c2): Chimeric nucleic acid comprising at least one of the nucleic acids selected from the group consisted of the above-described (a1) - (a5) and at least one of the nucleic acids selected from the group consisted of the above-described (b1) - (b6).
(c3) : Nucleic acid consisted of a nucleotide sequence in the above-described (c1) and (c2) in which one or several nucleotides are deleted, replaced or added, and which encodes for a polypeptide having functions of both human PTB domain and human ERK2 binding domain.

The signaling inhibitor in this embodiment may be one which comprises such nucleic acid as mentioned previously. And, according to the method for introducing the above-described nucleic acid into the target cell, for example, additional substance may be included as appropriate. The above-described method for introducing the nucleic acid is not particularly limited, and the method known in the prior art can be used. A specific example includes, for example, a method of utilizing a vector and a method without using a vector.

In the case where the above-described vector is used, it is preferable that, for example, the above-described signaling inhibitor further comprises an additional vector, and the above-described nucleic acid is linked to the above-described vector. The above-described vector is not limited, but any vector known in the prior art can be used. The above-described vector includes, for example, a nonviral vector and a viral vector, and it is preferable to select a vector which is capable of expressing the above-described nucleic acid in a target cell or in a target living body. The above-described nonviral vector includes, for example, expression vectors such as pGEX-4T-1, pcDNA3.1 (Invitrogen), pZeoSV (Invitrogen), pBK-CMV (Stratagene) and pCAGGS. In addition, the viral vector includes, for example, retroviral vector, lentivirus vector, adenovirus vector, adeno-associated virus vector (AAV vector), DNA virus and RNA virus such as herpesvirus, Vaccinia virus, poxvirus, poliovirus, sindbisvirus, Sendai virus, SV40 and immunodeficiency virus (HIV). Generally, the above-described nucleic acid may be inserted into the downstream of promoter of these vectors to make the expression thereof possible. In this manner, a recombinant vector produced by insertion of the above-described nucleic acid into a vector can be used as an above-described signaling inhibitor.

The above-described recombinant vector may further comprise a regulatory sequence which regulates expression of the above-described gene. The above-described regulatory sequence includes, for example, constitutive promoters such as cytomegalovirus (CMV)-derived promoter, Rous sarcoma virus (RSV), simian virus-40 (SV-40), muscle β-actin promoter and herpes simplex virus (HSV); tissue-specific promoter such as thymidine kinase promoter; inductive promoter and controlling promoter such as growth-hormone controlling promoter, promoter under regulation of lac operon sequence and zinc-inductive metallothionein promoter. The above-described regulatory sequence may be allocated or coupled to a site where the expression of the above-described gene can be controlled functionally based on the method known in the prior art. In addition to this, for example, enhancer sequence, polyadenylation signal and the sequence of the replication origin (ori) may be comprised.

In addition, the above-described recombinant vector may have a sequence which encodes for a selection marker such as, for example, drug-resistance marker, fluorescent protein marker, enzyme marker, cell-surface receptor marker, and the like.

In the case of a method in which the vector is not used, it is preferable for the above-described signaling inhibitor to comprise further, for example, liposome and particles (metal particles) for gene gun. For example, by incorporating the above-described nucleic acid into the liposome, the nucleic acid in the above-described liposome can be incorporated into the cell. In addition, for example, by shooting the above-described particle coated with the above-described nucleic acid into the cell by a gene gun, the above-described nucleic acid can be transported into the cell. In such case, to make the above-described nucleic acid possible to express the objective polypeptide in the cell, it is preferable to comprise further, for example, regulatory sequence such as promoter.

The method for introducing the signaling inhibitor including the above-described nucleic acid into test subj ects is not limited. There are, for example, a method of introducing in vivo the signaling inhibitor directly into the body, and a method in which the above-described signaling inhibitor is introduced ex vivo into the target cell or tissue derived from the test subject, and the cell and the like which has been incorporated with the signaling inhibitor is returned to the body of the test subject.

In the case of the former in vivo method, for example, after an appropriate sterilization treatment, the above-described signaling inhibitor may be introduced into the test subject. The method of introduction is not particularly limited, and the method known in the prior art such as, for example, administration by injection or gene gun, liquid immersion can be employed as appropriate.

In the case of the latter ex vivo method, for example, calcium phosphate method, polyethylene glycol method, lipofection method, electroporation method, ultrasound nucleic acid transfer method, method of introduction by gene gun, DEAE-dextran method, direct infusion method using glass capillary, method using virus vector as mentioned previously are included.

As stated above, if the signaling inhibitor including the above-described nucleic acid is administered into the cell, the polypeptide as mentioned previously will be expressed in the cell. By the expressed polypeptide, the signaling pathway of ErbB2 will be down-regulated.

### Second embodiment (administration of polypeptide)

The method for inhibiting signaling of the present invention comprises a step of administration of a signaling inhibitor including the above-described polypeptide. As stated above, by the direct administration of the above-described polypeptide into the target cell, the signaling pathway of ErbB2 can also be down-regulated. In this regard, hereinafter, the signaling inhibitor including the above-described polypeptide is sometimes-referred to as "the second signaling inhibitor" of the present invention.

The above-described polypeptide may be, for example, naturally-occurring polypeptide, or synthesized polypeptide by chemical synthesis. From the viewpoint that easy preparation on a large scale is possible, polypeptides or proteins which can be expressed through the use of recombinant DNA technology is preferable. The method for preparing polypeptide using such recombinant DNA technology is not particularly limited, however, for example, the recombinant vector as mentioned in the above-described first signaling inhibitor may be used. For example, based on a host-vector system, the above-described recombinant vector is introduced into a suitable host, and thereby, the above-described polypeptide can be expressed. The polypeptide obtained by such method is, if necessary, subjected to purification treatment such as, for example, salt precipitation, centrifugal separation and column chromatography, the purified polypeptide recovered can be used as a signaling inhibitor of the present embodiment.

The method for administering the signaling inhibitor including the above-described polypeptide into test subjects is not particularly limited, and the method known in the prior art can be employed. For example, any method can be employed as appropriate depending on the type of the target cell or tissue, and includes, for example, injection, liquid immersion, and the like.

The above-described signaling inhibitor may further comprise liposome or polymer, and the above-described polypeptide may be encapsulated in the above-described liposome, or the above-described polypeptide may be coupled with the above-described polypeptide.

As stated above, by administration of the signaling inhibitor including the above-described polypeptide into the cell, the signaling pathway of ErbB2 can be down-regulated.

In the method for inhibiting signaling of the present invention, target human cell is not limited; however, the method is useful for, for example, the cells with overexpression of ErbB2. In the present invention, expression of ErbB2 comprises transcription of erbB2 gene to RNA and expression of ErbB2 protein. From the fact that the overexpression of ErbB2 is linked with carcinogenesis, it is effective to apply the present invention to the cancer cell such as, for example, breast cancer, ovarian cancer, gastric cancer, bladder cancer, oral cancer, esophageal cancer, brain tumor, lung cancer, craniocervical cancer, skin cancer, uterus cancer, colon cancer, pancreas cancer. Among them, particularly in the breast cancer, overexpression of ErbB2 is known to be involved in the carcinogenesis, and therefore, the present invention is effective for application to the breast cancer cell. In addition, application to the cells which have the potential of developing the above-described cancers, for example, normal cell of breast, ovary, stomach, bladder, oral cavity, esophagus, brain and lung, craniocervical region, skin, uterus, colon, pancreas is also useful. In addition, the target human cell may be the cells collected from living body, or also the cells in the living body.

According to such method for inhibiting signaling of the present invention, signaling of cell can be inhibited through ErbB2 in the cell. In this way, for example, malignant alteration of the cell by activation of signaling pathway of ErbB2, or proliferation of the cell (e.g. cancer cell) can be restrained. It should be noted that the present invention can also be applied to animal and animal cell other than humans (hereinafter, same as above).

### <Signaling inhibitor>

The signaling inhibitor of the present invention is, as mentioned previously, the inhibitor which is capable of inhibiting activation of the signaling pathway mediated by ErbB2 in a human cell. The signaling inhibitor of the present invention includes, firstly, as mentioned previously, the first signaling inhibitor comprising nucleic acid. The above-described first inhibitor may comprise a nucleic acid which encodes for at least one of polypeptide having a function of the above-described PTB domain and a polypeptide having a function of ERK2 binding domain, and expresses it in the cell. The first signaling inhibitor comprising such nucleic acid is as mentioned previously. Next, the signaling inhibitor of the present invention includes the second signaling inhibitor comprising the polypeptide as mentioned previously. The above-described second inhibitor may comprise at least one of polypeptide having a function of the above-described PTB domain and a polypeptide having a function of ERK2 binding domain. The second signaling inhibitor comprising such polypeptide is as mentioned previously.

According to the signaling inhibitor of the present invention, signaling of cell can be inhibited. In this way, for example, malignant alteration of the cell by activation of signaling pathway mediated by ErbB2, or proliferation of the cell (e.g. cancer cell) can be restrained. Therefore, the signaling inhibitor of the present invention is useful, for example, as an anticancer drug or a cell proliferation inhibitor.

### <Method for treating cancer>

Method of treatment of the present invention is a method for treating human cancer, and comprises a step of administration of the signaling inhibitor of the present invention. The method of treatment of the present invention is characterized by the use of the signaling inhibitor of the present invention itself, and other processes and conditions are not limited. The method for administering the signaling inhibitor of the present invention is, for example, the same as mentioned previously. Specifically, the above-described signaling inhibitor may be directly introduced, in vivo into the malignant transformation part of the human, and a method may be given in which the above-described signaling inhibitor is introduced, ex vivo, into the target cell or tissue taken out, and the cell and the like after introduction is returned into the human body after the introduction. In addition, the cancer to which the present invention is applied preferably is, for example, the one in which the signaling pathway of ErbB2 is involved, and, for example, breast cancer, ovarian cancer, gastric cancer, bladder cancer, oral cancer, esophageal cancer, brain tumor, lung cancer, craniocervical cancer, skin cancer, uterus cancer, colon cancer, pancreas cancer and the like are included. In addition, application to the cells having a potential of developing the above-described cancer, for example, the normal cell of breast, ovary, stomach, bladder, oral cavity, esophagus, brain, lung, craniocervical region, skin, uterus, colon, pancreas region and the like is also useful. It should be noted that the method of treatment of the present invention also comprises, for example, prevention of malignant alteration.

### <Anticancer drug>

Anticancer drug of the present invention is an anticancer drug for human use, and comprises the signaling inhibitor of the present invention. The anticancer drug of the present invention may comprise the above-described signaling inhibitor, and its form is not limited. In addition, method of its usage and the like are also the same as described above.

As mentioned previously, it has been known that the overexpression of ErbB2 which are receptors of signaling pathway is involved in the malignant alteration. Therefore, the anticancer drug comprising the signaling inhibitor of the present invention is useful for the treatment of the cells which overexpress ErbB2, or cancer cells in which these signaling pathways are involved. The above-described cancer cells include, for example, breast cancer, ovarian cancer, gastric cancer, bladder cancer, oral cancer, esophageal cancer, brain tumor, lung cancer, craniocervical cancer, skin cancer, uterus cancer, colon cancer, pancreas cancer and the like. In addition, application to cell which has a potential of developing the above-described cancer, for example, normal cell of breast, ovary, stomach, bladder, oral cavity, esophagus, brain, lung, craniocervical region, skin, uterus, colon, pancreas region and the like is also useful. It should be noted that the anticancer drug of the present invention also includes, for example, preventive agent for malignant alteration.

The anticancer drug of the present invention may include further the anticancer drug known in the prior art. Herceptin, Iressa (gefitinib), Tarceva (erlotinib), sunitinib, lapatinib and the like may be included as the above-described anticancer drug.

According to the research by the present inventors, it is revealed that the inhibitory effect of signaling by the above-described monoclonal antibody is promoted, when the signaling of ErbB2 is down-regulated by FRS2β. Consequently, the signaling inhibitor of the present invention can be said to be a promotor of the drug efficacy of anti-cancer drug of known in the prior art. Herceptin, Iressa (gefitinib), Tarceva (erlotinib), sunitib, lapatinib, and the like may be included as the above-described anticancer drug. In such cases, for example, the promotor of the drug efficacy of the present invention may be used in combination with anti-cancer drugs of known in the prior art, and treatment or prevention can be performed by the precedent administration of the promotor of the drug efficacy of the present invention, followed by the administration of the anti-cancer drugs of known in the prior art.

### <Cell proliferation inhibitor>

Cell proliferation inhibitor of the present invention comprises the signaling inhibitor of the present invention. The cell proliferation inhibitor of the present invention may comprise the above-described signaling inhibitor, and its form is not limited. In addition, method of its usage and the like are also the same as described above. In addition, the cells to which the cell proliferation inhibitor of the present invention is applied are not limited; however, the cells include the cancer cells or normal cells as described above.

### <Marker>

Marker of the present invention is, as mentioned previously, the marker for determining presence of down-regulation in the signaling pathway, wherein the above-described signaling pathway is mediated by ErbB2 in a human cell; and the marker comprises at least one of human FRS2β and transcription product of FRS2β gene.

As mentioned previously, it has been clarified by the inventors that human FRS2β down-regulates signaling pathway of ErbB2. Therefore, by determining the presence of expression or the expression level of human FRS2β in the target human cell, judgment on whether the above-described signaling pathway is down-regulated or not can be made. The similar judgment can also be made by the presence of transcript or transcript level (e.g., amount of mRNA) of human FRS2β gene. In a specific example, for example, using the FRS2β-mRNA / GAPDH-mRNA value of 1, 452 x 10⁻² as a cutoff value, the judgment on the presence of down-regulation can be made.

Further, by making judgment on the presence of down-regulation, the following things will become possible.
(1) As mentioned previously, it has been known that the overexpression of ErbB2 and the activation of overexpressed ErbB2 plays a role in the malignant alteration. Therefore, if detection of the marker of the present invention in a human cell is carried out and judgment on the presence of down-regulation is made, for example, risk of malignant alteration of the human cell can be determined. That is, when the down-regulation is taken place, it can be determined that possibility of malignant alteration attributable to the above-described signaling pathway is low; and when the down-regulation is not taken place, it can be determined that there is a possibility of malignant alteration attributable to the above-described signaling pathway.
(2) If the detection of the marker of the present invention for human cell is carried out and judgment on the presence of down-regulation is made, it can be determined whether the malignant alteration of the above-described cancer cells is attributable to the above-described signaling pathway or attributable to other mechanism.
(3) Further, as stated in the above (2), if the judgment on whether the cause of the malignant alteration is attributable to the above-described signaling pathway is made, therapeutic strategy of the cancer can also be decided. As an example, the therapeutic strategy of breast cancer will be described.
   In about 10 - 30% of breast cancer patients, overexpression of ErbB2 has been identified, and as an effective anticancer drug for such patients, monoclonal antibody such as the above-described Herceptin which is capable of inhibiting the function of ErbB2, has been used. However, it has been a subject of discussion that Herceptin is effective in less than about 40% of the patients, even if ErbB2 is strongly positive. In this time, the research by the present inventors showed that the inhibitory effect of signaling was promoted by the above-described monoclonal antibody when the signaling of ErbB2 has already been down-regulated by FRS2β. And so, detection of the marker of the present invention for the breast cancer cell of the patient and determination of the presence of down-regulation are carried out, and thereby, judgment can be made on whether the treatment by low molecular weight compound such as Harceptin is appropriate.
(4) In addition, with respect to breast cancer, Hercep Test which detects ErbB2 overexpression as mentioned previously has been carried out. However, while phosphorylation of ErbB2 is needed to be detected in this test, other ErbB family is also detected. Therefore, there remains a problem of difficulty in determining whether the signaling of ErbB2 is actually working. In contrast to this, if the marker of the present invention is detected and determination of the presence of down-regulation is carried out, it is possible to detect whether the signaling of ErbB2 is actually working, instead of ErbB2 itself.

The amino acid sequence of human FRS2β and the nucleotide sequence of human FRS2β gene have been registered in NCBI Accession No. MN_006653. As the above-described marker, for example, transcription product (mRNA) of human FRS2β gene is preferable, and measurement on presence of the transcript of the above-described mRNA, or the amount of the transcript is preferable.

### <Method for measuring marker>

Measurement of the marker of the present invention is not limited, and can be determined suitably depending on the type of the marker.

### (1) Measurement of human FRS2β [0078]

When the above-described marker of the present invention is human FRS2β, for example, methods of measurement known in the prior art for detection of particular polypeptide or protein can be employed. The above-described method of measurement is not limited in any way. Specific example includes, for example, immunoassay method using antibody. The above-described immunoassay method includes, for example, enzyme-linked immunosorbent assay (ELISA) method, immunoagglutination method such as latex immunoagglutination method, immunonephelometry such as latex immunonephelometry, radioimmunoassay, and western blotting method. Among them, as an above-described immunoassay method, ELISA is preferable. The above-described ELISA includes, for example, sandwich ELISA method and competitive ELISA method.

In the case where human FRS2β is measured by immunoassay, an antibody for detection includes, for example, anti-human FRS2β antibody. The above-described anti-human FRS2β antibody may be prepared, for example, by the method known in the prior art. To give a specific example, for example, an animal is immunologically sensitized by inoculation of human FRS2β as an antigen, polyclonal or monoclonal anti-human FRS2β antibody can be obtained. Animal species of host cell to be sensitized immunologically is not particularly limited, and for example, human, mammal other than human such as rabbit, rat, mouse, goat, sheep, horse, pig, guinea pig, and the like, avian species such as chicken, pigeon, duck, quail, and the like can be used. In addition, the method for inoculating antigen to an animal is also not particularly limited and intradermal administration, subcutaneous administration, intraperitoneal administration, intravenous administration, intramuscular administration, and the like can be employed. The class of immunoglobulin about the antibody obtained in this way is generally IgM and IgG. The antibody obtained can be used, for example, as it is, and further, active fragment of the antibody such as Fab, Fab' , F(ab')₂ obtained by enzymatic treatment can also be used as an antibody.

The measurement of human FRS2β will be explained by taking an example. It should be noted that the present invention is not limited thereto.

That is, the method for measuring the marker of the present invention comprises the following steps (1) and (2), wherein the above-described marker comprises FRS2β, as mentioned previously:
(1) the step in which, anti-human FRS2β antibody against the above-described human FRS2β is added to a biological sample, and then the above-described human FRS2β in the above-described biological sample is allowed to bind with the above-described anti-human FRS2β antibody to form a complex;
(2) the step of measurement of the above-described complex.

Due to excellence in handling, the above-described antibody is preferably fixed (adsorbed) onaplate (forexample, ELISA plate), for example, prior to the above-described step (1). In this case, by adding a biological sample to the above-described plate, the above-described complex can be formed in the above-described plate. In the above-described step (2), the method for measuring the above-described complex is not particularly limited. To give a specific example, a method of measurement in which the antigen (human FRS2β in the biological sample) in the above-described complex is further bound with a labeled anti-human FRS2β antibody, and then the above-described labeled antibody is measured, is included (sandwich immunoassay method). The above-described labeling is not particularly limited, and includes, for example, the labeling known in the prior art such as enzyme labeling, fluorescence labeling and radioisotope labeling. Among them, as a labeled antibody, enzyme-labeled antibody is preferable.

An example of sandwich ELISA method using enzyme as a labeling substance will be explained. First, human cell to be a target of diagnosis is collected. And, protein is extracted from the above-described cell to prepare extraction fraction. On the other hand, anti-human FRS2β antibody against human FRS2β of measurement object is prepared, and then immobilized to a measuring container. Then, the above-described extraction fraction is added to the above-described measuring container. Thereby, the immobilized antibody is reacted with the antigen (human FRS2β) in the above-described extraction fraction, and both of them are coupled. After washing the above-described measuring container, the enzyme-labeled antibody (labeled anti-human FRS2β antibody) is added. In this way, the antigen coupled to the above-described immobilized antibody is reacted with the above-described labeled antibody, and both of them are coupled, and thereby a complex with a configuration in which the above-described antigen is sandwiched between two antibodies is formed. And, after the labeled antibody which is not coupled with the above-described antigen is removed, activity of the labeling substance (enzyme) in the above-described complex is measured. This enzyme activity is proportional to the amount of the above-described complex, and in consequence, this shows the amount of antigen of the measurement obj ect in the above-described extraction fraction. The enzyme described above is not limited in any way, and, for example, peroxidase, alkaline phosphatase or β-galactosidase can be used. The two kinds of antibodies used in this method preferably have, for example, different binding sites from each other for the same antigen.

### (2) Measurement of transcription product (mRNA) of human FRS2β [0084]

In the case where the above-described marker of the present invention is transcription product of human FRS2β, for example, the presence of the transcript mRNA or transcription level may be measured. For the method of such measurement, those known in the prior art can be employed. Specific example includes, for example, a nucleic acid amplification method using specific primer and a method using detection probe. The former includes, for example, PCR method, reverse transcription PCRmethod and real-time PCR; the latter includes, for example, Southern blotting method and Northern blotting method. It should be noted that, the sequence of the specific primer and the detection probe can be decided as appropriate based on the sequence of human FRS2β gene and mRNA.

In the case where the transcript mRNA is measured, generally, the reverse transcription PCR method or the real-time PCR method has been widely used. In these procedures, for example, cDNA is synthesized by the reverse transcription PCR method using total RNA or mRNA expressed in a biological sample as a template, and then the objective sequence is amplified by the real-time PCR method using the above-described cDNA as a template. In this way, amount of the objective mRNA which has been expressed in the above-described biological sample can be measured. Therefore, in the present invention, for example, in addition to the mRNA of human FRS2β gene expressed in a biological sample, cDNA synthesized by genetic engineering procedure such as PCR and amplified objective sequence (amplified DNA product) can also be referred to as the marker. In this regard, the cDNA to be synthesized using total RNA or mRNA as a template is not particularly limited, and may comprise, for example, an entire length of cDNA of human FRS2β gene or a partial sequence thereof. In addition, the objective sequence to be amplified using cDNA as a template may be, for example, an entire length of cDNA for human FRS2β, or a partial cDNA sequence.

### <Measurement kit for marker>

The kit for measuring the marker of the present invention includes a first kit for measuring the marker comprising the above-described human FRS2β, and a second kit for measuring the marker comprising a transcript of the above-described human FRS2β gene. The above-described first kit comprises antibody specific for human FRS2β. The above-described antibody is the same as mentioned previously. In addition, the second kit comprises at least one selected from the group consisted of a probe specific for human FRS2β gene and a primer specific for human FRS2β gene. The above-described probe and primer are the same as mentioned previously. The measuring kit for marker of the present invention may include further the instruction manual.

As mentioned previously, by detecting the marker of the present invention, the down-regulation of signaling pathway can be determined. Therefore, the kit for measuring the marker of the present invention can also be referred to as a kit for determining presence of down-regulation of signaling pathway mediated by ErbB2 in a human cell. In addition, the kit for measuring the marker of the present invention can also be referred to as a kit for determining, from presence or absence of down-regulation, necessity of treatment for human cancer cell with an anticancer drug. The above-described anti-cancer drug is not limited in any way, for example, the drugs known in the prior art, such as Herceptin, Iressa (gefitinib), Tarceva (erlotinib), sunitinib, lapatinib, and the like may be included as the above-described anticancer drug.

### <Method for diagnosis>

The method for diagnosis of the present invention is to determine necessity of treatment for human cancer cell with and anticancer drug comprising, which comprises a step of detecting the marker of the present invention. As mentioned previously, for a cancer in which overexpression of ErbB2 is causative, for example, Harceptin and the like have been used as a molecular-targeted anticancer drug. However, as stated previously, it has been become a subject of discussion that Herceptin is effective in less than about 40% of the patients, even if ErbB2 is strongly positive. According to the research by the present inventors, it is revealed that the inhibitory effect of signaling by the above-described monoclonal antibody is promoted, when the signaling of ErbB2 is down-regulated by FRS2β. Therefore, in the present invention, detection of the marker and the determination of the presence of down-regulation are carried out, and thereby, judgment on the necessity of treatment with molecular-targeted anticancer drug can be made.

### <Use of nucleic acid and polypeptide>

The present invention is use of at least one nucleic acid selected from the groups consisted of the above-described (a1)-(a5) and (b1) - (b6) for inhibiting the activation of signaling pathway mediated by ErbB2 in a human cell. In addition, the present invention is use of at least one nucleic acid selected from the group consisted of the above-described (a1) - (a5) and (b1) - (b6) for treating human cancer. In addition, the present invention is use of at least one polypeptide selected from the group consisted of above-described (A1)-(A3) and (B1) - (B4) for inhibiting the activation of signaling pathway mediated by ErbB2 in a human cell. In addition, the present invention is use of at least one polypeptide selected from the group consisted of the above-described (A1)-(A3) and (B1) - (B4) for treating human cancer.

In the next place, an Example of the present invention will be described. In this regard, however, the present invention is not limited in any way by the following example.

### EXAMPLE 1

### (Preparation of recombinant vector)

The entire length of cDNA of FRS2β and the code sequence of FLAG-tag, which adds the FLAG at C terminus, were linked to retroviral vector (pMXs-puro vector: supplied by Professor Toshio Kitamura, Institute of Medical Science, university of Tokyo), plasmid vector (commercial name, pcDNA: Sigma Biochemical Corporation)and Lentivirus (CSII-EF vector, supplied from the Bioresource Center, RIKEN). These are referred to FRS2β expression retroviral vector, FRS2β expression plasmid vector and FRS2β expression lentivirus vector. The objective polypeptides expressed by these vectors are added to FLAG-tag at their C-termini. In addition, cDNA encoding for Δ232 -252 FRS2β deleted the 232nd - 252nd region in amino acid sequence of FRS2β shown in SEQ ID NO: 1, was prepared, and in the same manner, was linked to the above-described retroviral vector, the above-described plasmid vector or the above-described lentivirus vector. These are referred to Δ232 -252 FRS2β expression retroviral vector, Δ232 -252 FRS2β expression plasmid vector, and Δ232 -252 FRS2β expression lentivirus vector.

The entire length of cDNA of the human wild type ErbB2 was prepared according to the accession No. NM_004448 (human erbB2) of NCBI database. And the above-described entire length cDNA is linked to plasmid vector (commercial name, pc-DNA-neo: Sigma Biochemical Corporation). The expression vector linked with wild type ErbB2 cDNA is referred to wild type ErbB2 expression plasmid vector. It should be noted that, the vector which is not linked with the above-described cDNA was used as control vector.

An entire length of cDNA of human active type ErbB2 with abnormal mutation in membrane-spanning domain was prepared according to database accession No. NM_004448 (human erbB2). The above-described mutation is mutated at the 659th amino acid valine replaced by glutamic acid, in the wild type ErbB2. And, the above-described entire length of cDNA was linked to the above-described plasmid vector. The expression vector linked with cDNA of the active type ErbB2 is referred to as activated type ErbB2 expression plasmid vector.

A cDNA of ErbB2 mutant was prepared by introduction of the mutant corresponding to the mutant in membrane-spanning domain, which exists in rat breast cancer gene, into cDNA of human wild type ErbB2. The above-described mutation is mutated at the 659th amino acid valine replaced by glutamic acid in human wild type ErbB2. And, the-described entire length of cDNAwas linked to the above-described plasmid vector. The expression vector linked with the cDNA of mutant type ErbB2 is referred to as mutant type ErbB2 expression plasmid vector or active type ErbB expression plasmid vector.

A cDNA encoding for the entire length of human ErbB3 was prepared according to NM_001982 (human erbB3). And, the above-described cDNA was linked to the same plasmid vector as previously stated. The expression vector linked with the cDNA of ErbB3 is referred to as ErbB3 expression plasmid vector.

### 1. Suppression of transforming activity by FRS2β

The suppression of transforming activity by FRS2β was determined by expressing the ErbB2 and FRS2β in NIH3T3 cell.

### (Colony forming assay in soft agar)

A stable transformant was obtained by transfection of NIH3T3 cell by using the above-described active type ErbB2 expression plasmid vector (reference literature: BBRC 178, p724-732, 1991). Into this cell, FRS2β expression retroviral vector, Δ232-252 FRS2β expression retroviral vector and blank retroviral vector were introduced, respectively, and treated with puromycin, and the stable transformant was selected. These transformants were suspended in DMEM medium containing 10% newborn calf serum (NBCS) and 3% agarose. Subsequently, this suspension (number of cells: approximately 10000) was piled up on the DMEM medium containing 0.6% agarose on a culture plate of 3. 5 cm diameter. And, the medium was cultured under the condition of 5% CO₂ at 37°C, after 3 weeks, colonies visible with naked eyes (number of colonies having around 0.2 mm or more in diameter and visible with naked eyes) were counted. It should be noted that, as a control, the same assay was performed with the NIH3T3 cells which express activated ErbB2 with no introduction of the retroviral vector.

The results of the assay are shown in Figs. 1 and 2. Fig. 1 shows photographs representing morphology of the activated ErbB2 expression NIH3T3 cell in which no expression retroviral vector was introduced and the activated ErbB2 expression NIH3T3 cell in which expression retroviral vector was introduced. In the same Figure, photograph at the top is a photograph representing morphology of the active ErbB2 expression NIH3T3 cell in which no expression retroviral vector was introduced. The photographs at the bottom show morphologies representing the active ErbB2 expression NIH3T3 cell in which various expression retroviral vectors were introduced; left is the results of cells in which only blank retroviral vector was introduced (control), middle is the results of cells in which FRS2β expression vector was introduced, and right is the results of cells in which Δ232-252FRS2β expression vector was introduced. Fig. 2 shows a graph representing the number of colonies of the above-described each cell. In the same Figure, vertical axis shows numbers of colonies formed from culture of 5 x 10⁴ cells. In the same Figure, "Control" indicates the results of the cells introduced with only the blank retroviral vector, "FRS2β" indicates the results of cells introduced with the FRS2β expression vector, and "Δ232-252FRS2β" indicates the results of cells introduced with the Δ232-252FRS2β expression vector.

As shown in the both Figures, transformed colonies were observed from the cells in which only active type ErbB2 was expressed, or cells in which only blank retroviral vector was introduced. Comparing with this, the number of transformed colonies was less in the cells in which FRS2β was expressed. In addition, in the cells expressed by Δ232/252FRS2β deleted the 232nd - 252nd region of FRS2β, recovery in the number of transformed colonies was observed. From this result, it was revealed that the transforming activity by ErbB2 was suppressed by the expression of FRS2β, and in Δ232/252FRS2β lacking the 232nd - 252nd region, the inhibitory effect of transforming activity is attenuated compared with the wild type FRS2β.

### 2. Suppression of proliferation of human breast cancer cell by FRS2β

FRS2β was expressed in human breast cancer MCF-7 cells and the suppression of proliferation was confirmed.

FRS2β expression retroviral vector was introduced into MCF-7 cell by the same way as above-described "1.", and stable transformant was obtained. This transformant cells were cultured in 0.5% fetal bovine serum (FBS) containing DMEM, under the condition of 5% CO₂, at 37°C and under the presence or absence of EGF (10 ng/ml). And, the number of cells was counted at the predetermined time (day) after the stimulating by the above-described EGF stimulation. In addition, as a control, the same proliferation assay was performed on the MCF-7 (mock) cell in which control vector was introduced.

The results are shown in Fig. 3. The Figure is a graph representing the changes in proliferation of the transformant MCF-7 cell with time. In this Figure, abscissa axis indicates days, and vertical axis indicates number of cells (x 10⁴). In this Figure, "FRS2β EBS+" indicates a result of transformant in which FRS2β was introduced and stimulated with EGF, and "FRS2β EGF-" indicates a result of transformant in which FRS2β was introduced but not stimulated with EGF, "mock EGF+" indicates a result of control stimulated with EGF, and "mock EGF-" indicates a result of control not stimulated with EGF. As shown in "FRS2β EGF+" in the Figure, the proliferation of human breast cancer cell line MCF-7 cell stimulated with EGF and serum was significantly inhibited by the expression of FRS2β.

### 3. Binding affinity of FRS2β for ErbB2

Determination on whether FRS2β binds to ErbB2 was carried out.

Wild type ErbB2 expression plasmid vector, activated type ErbB2 expression plasmid vector, FRS2β expression plasmid vector, and control vector were introduced transiently into HEK293T cell, respectively. These transformants were cultured in 10% FBS containing DMEM medium under the condition of 5% CO₂ at 37°C. Resultant cultured cells were solubilized with TNE buffer. The recovered solubilized solution was incubated with antibody (anti Flag M2 monoclonal antibody: Sigma-Aldrich) for 1 hour at 4°C, after that, protein G-Agarose beads (Produced by Amersham Biosciences) were added and incubated for 2 hours at 4°C. Supernatant was removed by centrifugation, the recovered above-described beads was washed 5 times with the above-described TNE buffer. After the washing, 2% dodecyl sulfate containing sample buffer was added to the above-described beads and was boiled for 3 minutes, and the resulting solution was applied to the sample for immunocoprecipitation experiment. And, the above-described sample was used for dodecyl sulfate polyacrylamide gel electrophoresis (SDS-PAGE), and immunoblotting was performed with antibody. As the antibody of the immunoblotting, anti-EGFR antibody (produced by Santa Cruz Biotechnology, Inc.) and anti-ErbB2 antibody (OP15, produced by Calbiochem) were used.

The results are shown in Fig.4. The Figure shows the photographs representing the results of immunoblotting of HEK293T cells expressing the ErbB2 and FRS2β. In the Figure, from the top, the first block is a band of ErbB2, the second block is a result of immunoblotting representing a band of FRS2β, and the third block is a result of electrophoresis of the solubilized solution. In the ErbB2 lane of the Figure, "-" indicates no introduction of ErbB2 expression vector, "wt" indicates introduction of wild type ErbB2 expression vector, "VE" indicates introduction of activated type ErbB2 expression vector, and in the lane of FRS2β, "-" indicates no introduction of FRS2β expression vector "+" indicates introduction of FRS2β expression vector. As shown in the Figure, it was confirmed that the FRS2β expressed in HEK293T cell can bind with the wild type ErbB2 and the activated type ErbB2.

### 4. Effect of FRS2β on ErbB2 and ErbB3

It has been known that ErbB2 forms heterodimer with ErbB3 of the same Erb family, and the intracellular domain of the above-described receptors (ErbB2 and ErbB3) are activated by the induction of the dimer. So, the effect of FRS2β on the activation of ErbB2 and ErbB3 was determined.

FRS2β expression retroviral vector and control vector were introduced into human breast cancer cell line MCF-7, respectively, and selected with puromycin, and the stable transformants were obtained. These transformants were cultured using DMEM containing 0.5% FBS, under the condition of 5% CO₂ at 37°C for 12 hours, and then stimulated for 10 minutes under the presence or absence of neuregulin (NRG: 10 ng/ml), which is a ligand of ErbB3. Sample for immunocoprecipitation was prepared according to the same process as above-described "3." except for using anti-ErbB2 antibody (OP15, Calbiochem) and anti-ErbB3 antibody (sc-285G, Santa Cruz Biotechnology, Inc), and immunoblotting was performed using the antibody. For an antibody for immunoblotting, anti-phosphotyrosine antibody (4G10, Biotech) was used.

The results are shown in Fig. 5. The Figure shows the photograph representing the result of immunoblotting of MCF-7 cells introduced with various expression vectors. In the Figure, from the top, the blocks show the results of immunoblotting representing the bands of tyrosine-phosphorylated ErbB3, ErbB3, tyrosine-phosphorylated ErbB2 and ErbB2, and, the band at the bottom shows the result of electrophoresis of the solubilized solution. In the FRS2β lane of the Figure, "-" indicates no introduced of FRS2β expression vector, "+" indicates introduction of FRS2β expression vector, and in NRG lane, "+" indicates addition of NRG, "-" indicates no addition of NRG. As shown in the Figure, among the cells stimulated by NRG, the autophosphorylation (tyrosine phosphorylation) of ErbB3 and ErbB2 was suppressed more in Fry2β expressing cell (FRS2β+: NRG+) compared with the FRS2β non-expressing cell (FRS2β-: NRG+). From this result, it can be concluded that FRS2β can inhibit the activation (tyrosine phosphorylation) of ErbB2 and ErbB3, and thereby, signaling pathway of ErbB2 can be down-regulated.

### 5. Effects on the phosphorylation of ERK and EGFR

Effect of FRS2β on the phosphorylation of ERK and ErbB3 was determined.

FRS2β expression retroviral vector and control vector were introduced into human breast cancer cell line BT474, respectively, and was selected with puromycin, and the stable transformants were obtained. These transformants were cultured using DMEM containing 0.5% FBS under the condition of 5% CO₂ at 37°C for 12 hours, and was stimulated for predetermined times (0 min, 10 min, 30 min, 1 h, 3h and 6 h) under the presence or absence of neuregulin (NRG: 10 ng/ml), which is a ligand of ErbB3. And, the cell lysate was subjected to electrophoresis by SDS-PAGE and immunoblotting was performed using anti-phosphorylated-ERK antibody, anti-ERK antibody, anti-phosphorylated-EGFR antibody, anti-phosphorylated- ErbB3 antibody, and anti-SPRY2 antibody, as antibodies. In addition, immunoblotting was performed in the same manner for BT474 cell (mock) in which control vector was introduced.

The results are shown in Fig. 6. The Figure shows photographs representing the results of immunoblotting of BT474 introduced with each expression vector. In the Figure, from the top, the blocks are the results of immunoblotting exhibiting the bands of tyrosine-phosphorylated ERK1/2, ERK1, tyrosine-phosphorylated EGFR, tyrosine-phosphorylated ErbB3 and SPRY2. In the Figure, "mock" indicates the results of BT474 cell in which control vector was introduced, "FRS2beta" indicates the results of the BT474 cell in which FRS2β expression retroviral vector was introduced, and Time (hr) indicates the time for NRG stimulation.

As shown in the Figure, among the cells stimulated by NRG, the autophosphorylation (tyrosine phosphorylation) and the phosphorylation of ERK, and the tyrosine phosphorylation of ErbB3 were suppressed more in the FRS2β expressing cell compared with the control cell ("mock") in which FRS2β is not expressed. From this result, it can be concluded that FRS2β can down-regulate the signaling of ErbB3, which is a heterodimer partner of ErbB2, and the activity of ERK at the down-stream.

### 6. Effect of FRS2β on the heterodimerization

The effect of FRS2β on the formation of the heterodimer consisted of ErbB2 and EGFR was determined.

Wild type ErbB2 expression plasmid vector, mutant type ErbB2 expression plasmid vector, FRS2β expression plasmid vector and control vector were introduced transiently into HEK293T cell, respectively. After the culture of these transformants in 0.1% FBS containing DMEM medium under the condition of 5% CO₂ at 37°C for 12 hours, the transformants were stimulated for 10 minutes under the condition of the presence or absence of EGF (10 ng/ml). And, the sample for immunocoprecipitation was prepared according to the same process as the above-described "3.", except for the use of antibodies, anti-ErbB2 antibody (OP15, Calbiochem) and anti-EGFR antibody (sc-03, polyclonal antibody, Santa Cruz) as anitibody, and immunoblotting was performed with using the antibody. As the antibody of the immunoblotting, anti-ErbB2 antibody, anti-EGFR antibody and anti-Flag antibody (M2 monoclonal antibody: Sigma-Aldrich) were used.

The results are shown in Fig. 7. The Figure shows photographs representing the results of immunoblotting of HEK293T cell introduced with each expression vector. In this Figure, upper four blocks are, from the top, the results of immunoblotting showing the bands of EGFR coprecipitated with ErbB2, ErbB2, ErbB2 coprecipitated with EGFR, EGFR, and lower three blocks are the results of electrophoresis of the solubilized solutions. In the Figure, "EGF" and "-" show the presence or absence of EGF stimulus, and "+ and -", in the lanes of SNT-2-FLAG (FRS2β), MT-ErbB2 and WT-ErbB2, indicates whether FRS2β expression vector, mutant type (MT) ErbB2 expression vector and wild type (WT) ErbB2 expression vector is introduced into the cell or not, respectively. As shown in the Figure, in FRS2β expressing cell (FRS2β+: EGF+), the formation of heterodimer of EGFR and ErbB2 generated by EGF stimulation was suppressed compared with FRS2β non-expressing cell (FRS2β-: EGF+). From this result, it is suggested, for example, that FRS2β interacts with other ErbB family molecules and also inhibits the formation of hetero- or homodimer of the ErbB family molecules. In addition, because the ErbB family molecule is activated by the heterodimer formation, it is presumed that ErbB4 is also inhibited by FRS2β.

### 7. Effect of FRS2β on the susceptibility to Herceptin

Effect on the susceptibility to Herceptin by expression of FRS2β was determined.

### (MTS assay)

An FRS2β expression plasmid vector and control vector (which expresses ds Venus) were introduced into human breast cancer cell line BT474 cell, and a stable transformant was obtained. These transformants were spread on the 96 well culture plate so as to be approximately 1 x 10⁴ cells per well, and cultured under the condition of 5% CO₂ at 37°C, in 10% fetal bovine serum containing RPMI medium. After 24 hours, medium was replaced to the media containing a dilution series of Herceptin, and cultured further. Ten days after the replacement of media, the media were treated further with MTS assay reagent (Promega Corporation) according to the attached protocol, the absorbance of wells was measured at 490 nm.

The results are shown in Fig. 8. In the Figure, (A) is a Figure showing the results of sorting of the expression of d2Venus, which is a indicator of multiplicity of infection, by FACS, based on the indicator of the fluorescence emitted from d2 Venus; in the Figure, vertical axis indicates the number of cells and horizontal axis indicates the intensity of fluorescence of cells. In the Figure, (B) is a photograph showing the electrophoresis of the FRS2β expressing cells and the cells in which control vector was introduced; right lane shows FRS2β expression cells and left lane shows cells introduced control vector. In addition, in the Figure, (C) shows the graph indicating the relationship between Herceptin concentration and absorbance of cultured cells. In the Figure (C), vertical axis indicates absorbance and abscissa axis indicates concentration of Herceptin, expressing the units (mcrg) of the concentration as microgram per 1 ml of medium. In addition, in the Figure, d2V indicates d2Venus, and wt indicates the wild type FRS2β.

From the results of the Figure (A), it was revealed that the multiplicity of infection of the lentivirus vector used in the present experiment was almost 100%. And, from the Figure (B), it was revealed that the wild type FRS2β was expressed by the introduction of lentivirus vector. And, from the Figure (C), it was revealed that, susceptibility to the cell to Herceptin was increased by the expression of FRS2β. From the above results, it was revealed that, in the breast cancer cell, susceptibility to Herceptin was increased by the expression of FRS2β (SNT-2). Based on this result, for example, because the FRS2β positive cancer cell can be judged to have high susceptibility to Herceptin, it can be said that FRS2β is useful as a biomarker for predicting the effect to anti-cancer drugs.

### 8. Tendency of ErbB2 and FRS2β in breast cancer cell line

Expression of ErbB2 and FRS2β in breast cancer cell line was determined.

Thirty-five cell lines of breast cancer cell were cultured according to the same process as the above-described "3." and solubilized solutions were prepared. These solubilized solutions were subjected to SDS-PAGE, and immunoblotting was performed with antibodies. As the antibodies for immunoblotting, anti-FRS2β antibody (Oncogene, vol. 25, p6457, 2006), anti-ErbB2 antibody (OP15, produced by Calbiochem Inc) and anti-ERK1/2 antibody (Santa Cruz Biotechnology, Inc) were used.

The results are shown in Fig. 9. The Figure is a photograph representing the results of immunoblotting of each breast cancer cells, and abscissa axis is the number showing the species of the various cell lines. Accordingly, as shown in the Figure, there were many cell lines over-expressing ErbB2 even in human breast cancer cell lines, as those found in human breast cancer tissues. FRS2β expression was found also in several cell lines. However, FRS2β expression in the cells over-expressing ErbB2 was generally low, and a trend of inverse correlation was observed between the ErbB2 expression and FRS2β expression. From this fact, it was revealed that expression of FRS2β was suppressed in the cancer cells under the condition dependent on the ErbB2 cancer genes. That is, it was suggested strongly that ErbB2 signal was down-regulated by FRS2β.

### 9. Tendency of ErbB2 and FRS2β in various cancer cell lines

Expression of ErbB2 and FRS2β in various cancer cell lines was determined.

For a total of 108 cell lines including breast cancer (35 cell lines), and lung cancer, colon cancer, gastric cancer, prostate cancer, brain tumor, pancreatic cancer, esophageal cancer, craniocervical cancer, kidney cancer and the like, the amount of the transcription product of FRS2β and erbB2 was determined by microarray method. The results are shown in Fig. 10. The Figure (A) is a graph representing the plot of the amount of transcription product by FRS2β to the amount of erbB2 transcription product in 108 cell lines of the cells, and the Figure (B) is a graph representing the plot of the amount of FRS2β transcription product to the amount of erbB2 transcription product in the 35 cell lines of breast cancer cell. As shown in the Figure, an inverse correlation was observed between the amount of FRS2β transcription product and the amount of erbB2 transcription product. From this result, it was revealed that expression of FRS2β was suppressed not only in the breast cancer under the condition dependent on the erbB2 cancer genes, but also in various cancer cells. That is, it was suggested strongly that ErbB2 signal was down-regulated by FRS2β in various cancer.

### 10. Tendency of ErbB2 and FRS2β in patients with breast cancer

Confirmation of expression of FRS2β in cancer tissue of a patient with breast cancer was carried out, and comparison with the expression of ErbB2 in the same patient was carried out.

The breast cancer tissues having the following 5 histological types were collected from patients. For these tissues, Hercept test was carried out according to the protocol, and determined by 4 stages. On the other hand, for the same breast cancer tissue, determination of FRS2β expression was carried out by immunostaining using anti-FRS2β antibody. Specifically, first, the respective breast cancer tissue was formalin-fixed, and deparafinized, and then the tissues were treated with 10 mM citrate buffer (pH 6) for 20 minutes by autoclaving. After that, the post-treatment breast cancer tissues were incubated with 100 times dilution with PBS of the above-described anti-FRS2β antibody at room temperature for 1 hour. The post-incubation breast cancer tissues were then stained by immunostaining using LSABC kit (product name, produced by Dako Japan) according to the attached protocol. As a coloring agent, 3,3'-diaminobenzidine was used. In addition, nuclear staining was performed using hematoxyline. The results are shown in the following table.

**[Table 1]**

| Tissue type | ErbB2 HER2/neu | FRS2β SNT-2 |
|---|---|---|
| Sample 1 scirrhous carcinoma | 3+ | negative |
| Sample 2 papillotubular carcinoma | 2+ | negative |
| Sample 3 solid tubular carcinoma | 1+ | negative |
| Sample 4 invasive lobular carcinoma | 0 | positive |
| Sample 5 scirrhous carcinoma | 0 | positive |
| Sample 6 scirrhous carcinoma | 0 | positive |

As described above, the samples judged as involving ErbB2 overexpression by Hercept test (Samples 1-3) were FRS2β (-), and the samples judged as involving no ErbB2 overexpression (Samples 4-5) were FRS2β(+). Fromthisresult, it can be considered that the expression of FRS2β and the expression of ErbB2 are in inverse correlation. In addition, it can be considered that the expression of FRS2β is possible to play a role in amplification of ErbB by some sort of mechanism.

### INDUSTRIAL APPLICABILITY

As described above, according to the polypeptide of the present invention, the signaling pathway mediated by ErbB2 may be down-regulated. Therefore, for example, by introducing the polypeptide of the present invention or the nucleic acid capable of expressing the polypeptide of the present invention into human target cell, the above signaling in the above-described cell can be suppressed. Further, from the fact that the signaling of ErbB2 is involved in the development of cancer as described above, prevention of the development of cancer and also treatment of the cancer may be performed by way of inhibiting signaling according to the present invention. In addition, also from the fact that, by the above-described inhibition of signaling, malignant alteration of cell as well as proliferation of cell can be suppressed, it can be said that the present invention is useful for the treatment of cancer.

In addition, since signaling of ErbB2 is down-regulated by FRS2β, judgment whether the above-described signaling is active or not is enabled by the detection of FRS2β in the targeted cell. And, based on the result of the judgment, for example, necessity of the treatment with molecular targeted anti-cancer drug which inhibits the function of ErbB2 will be able to judge. As stated above, it can be said that the present invention is exceedingly useful art in the fields of, for example, medical treatment or molecular cytology.

## Claims

1. An anticancer drug comprising at least one polypeptide selected from the group consisting of polypeptides (A1), (B1), (A2), (B2) and (C1):
(A1): a polypeptide comprising an amino acid sequence comprising the 1st - the 185th region in the amino acid sequence shown in SEQ ID NO: 1;
(B1): a polypeptide comprising an amino acid sequence comprising the 232nd - the 252nd or the 237th - the 252nd region in the amino acid sequence shown in SEQ ID NO: 1;
(A2): a polypeptide comprising an amino acid sequence in which 1-4 amino acid residues are deleted, replaced or added in the amino acid sequence of the 1st - the 185th region in the amino acid sequence shown in SEQ ID NO: 1, and having a function of binding to human ErbB2 equivalent to PTB domain of human FRS2β;
(B2): a polypeptide comprising an amino acid sequence in which 1-4 amino acid residues are deleted, replaced or added in the amino acid sequence of the 232nd - the 252nd or the 237th - the 252nd region in the amino acid sequence shown in SEQ ID NO: 1, and having a function of binding with human ERK2; and (C1) a polypeptide consisting of the amino acid sequence shown in SEQ ID NO: 1;
for use in a method of treating cancer by administration of said anticancer drug to an individual in need thereof, wherein the cancer is **characterised by** overexpression of ErbB2 or is a cancer in which the signaling pathway mediated by ErbB2 is involved.

2. An anticancer drug for use according to claim 1, comprising at least one polypeptide selected from the group consisting of polypeptides (A1), (B1), (A2) and (B2) :
(A1): a polypeptide consisting of an amino acid sequence comprising the 1st - the 185th region in the amino acid sequence shown in SEQ ID NO: 1;
(B1): a polypeptide consisting of an amino acid sequence comprising the 232nd - the 252nd or the 237th - the 252nd region in the amino acid sequence shown in SEQ ID NO: 1;
(A2): a polypeptide consisting of an amino acid sequence in which 1-4 amino acid residues are deleted, replaced or added in the amino acid sequence of the 1st - the 185th region in the amino acid sequence shown in SEQ ID NO: 1, and having a function of binding to human ErbB2 equivalent to PTB domain of human FRS2β; and
(B2): a polypeptide consisting of an amino acid sequence in which 1-4 amino acid residues are deleted, replaced or added in the amino acid sequence of the 232nd - the 252nd or the 237th - the 252nd region in the amino acid sequence shown in SEQ ID NO: 1, and having a function of binding with human ERK2.

3. An anticancer drug comprising at least one nucleic acid encoding a respective polypeptide selected from the group consisting of polypeptides (A1), (B1), (A2) and (B2) as defined in claim 1 or 2 for use in a method of treating cancer by administration of said anticancer drug to an individual in need thereof, wherein the cancer is **characterised by** overexpression of ErbB2 or is a cancer in which the signaling pathway mediated by ErbB2 is involved.

4. An anticancer drug for use according to claim 3, comprising at least one nucleic acid selected from the group consisting of nucleic acids (a1), (b1), (a2) and (b2):
(a1): a nucleic acid comprising a nucleotide sequence comprising the 1st - the 555th region of the nucleotide sequence shown in SEQ ID NO: 2;
(b1): a nucleic acid comprising a nucleotide sequence comprising the 709th - the 756th region of the nucleotide sequence shown in SEQ ID NO: 2;
(a2): a nucleic acid comprising an nucleotide sequence in which 1-6 nucleotides are deleted, replaced or added in the nucleotide sequence of the 1st - the 555th region of the nucleotide sequence shown in SEQ ID NO: 2, and encoding for a polypeptide having a function of binding to human ErbB2 equivalent to PTB domain of human FRS2β; and
(b2): a nucleic acid comprising an nucleotide sequence in which 1-6 nucleotides are deleted, replaced or added in the nucleotide sequence of the 709th - the 756th region of the nucleotide sequence shown in SEQ ID NO: 2, and encoding for a polypeptide having a function of binding with human ERK2.

5. An anticancer drug for use according to claim 3, comprising at least one nucleic acid selected from the group consisting of nucleic acids (a1), (b1), (a2) and (b2):
(a1): a nucleic acid consisting of a nucleotide sequence comprising the 1st - the 555th region of the nucleotide sequence shown in SEQ ID NO: 2;
(b1): a nucleic acid consisting of a nucleotide sequence comprising the 709th - the 756th region of the nucleotide sequence shown in SEQ ID NO: 2;
(a2): a nucleic acid consisting of an nucleotide sequence in which 1-6 nucleotides are deleted, replaced or added in the nucleotide sequence of the 1st - the 555th region of the nucleotide sequence shown in SEQ ID NO: 2, and encoding for a polypeptide having a function of binding to human ErbB2 equivalent to PTB domain of human FRS2β; and
(b2): a nucleic acid consisting of an nucleotide sequence in which 1-6 nucleotides are deleted, replaced or added in the nucleotide sequence of the 709th - the 756th region of the nucleotide sequence shown in SEQ ID NO: 2, and encoding for a polypeptide having a function of binding with human ERK2.

6. An anticancer drug for use according to claim 4, wherein said nucleic acid (a1) is a nucleic acid comprising the nucleotide sequence of the 1st - the 555th in the nucleotide sequence shown in SEQ ID NO: 2, and said nucleic acid (b1) is a nucleic acid comprising the nucleotide sequence of the 709th - the 756th in the nucleotide sequence shown in SEQ ID NO: 2.

7. An anticancer drug for use according to claim 4, wherein the nucleic acids of said (a1) or (b1) is human FRS2β gene comprising the nucleotide sequence shown in SEQ ID NO: 2.

8. An anticancer drug for use according to any one of claims 3 to 7 comprising at least one of the nucleic acids of (a1) and (a2) and at least one of the nucleic acids of (b1) and (b2).

9. An anticancer drug for use according to any one of claims 3 to 8 comprising a chimeric nucleic acid comprising at least one of the nucleic acids of (a1) and (a2) and at least one of the nucleic acids of (b1) and (b2).

10. An anticancer drug for use according to any one of claims 3 to 9, wherein said nucleic acid further comprises a vector and said nucleic acid is linked to said vector.

11. Use of at least one polypeptide selected from the group consisting of polypeptides (A1), (B1), (A2) and (B2) as defined in claim 1 or at least one nucleic acid as defined in any one of claims 2 to 8 encoding a respective said polypeptide for the preparation of a medicament for the treatment of cancer, wherein the cancer is **characterized by** overexpression of ErbB2 or is a cancer in which the signaling pathway mediated by ErbB2 is involved.

12. An anticancer drug for use according to any one of claims 1 to 10 or use according to claim 11, wherein the cancer is selected from the group consisting of breast cancer, ovarian cancer, gastric cancer, bladder cancer, oral cancer, esophageal cancer, brain tumor, lung cancer, craniocervical cancer, skin cancer, uterus cancer, colon cancer and pancreas cancer.

13. A substance selected from an antibody specific for human FRS2β, a probe specific for human FRS2β gene and a primer specific for human FRS2β gene, for use in a method of diagnosing the necessity of treatment of a human cancer cell with an anticancer drug, wherein when down-regulation of FRS2β signaling is detected, treatment with an ErbB2-targeted anti-cancer drug is necessary.

14. An in-vitro method for determining the presence of down-regulation of a signaling pathway mediated by ErbB2 in a human cell, which method comprises a step of detecting a down-regulation marker comprising at least one of human FRS2β and transcription product of FRS2β gene, wherein detection of FRS2β indicates that the ErbB2 signalling pathway is down-regulated.

15. An *in vitro* method of diagnosis for determining necessity of treatment of cancer with an anticancer drug, which method comprises a step of detecting a marker comprising at least one of human FRS2β and transcription product of FRS2β gene, wherein when down-regulation of FRS2β signaling is detected, treatment with an ErbB2-targeted anti-cancer drug is necessary.

16. A method according to claim 14 or claim 15, wherein the marker is detected using an antibody specific for human FRS2β, a probe specific for human FRS2β gene and a primer specific for human FRS2β gene.

## Patentansprüche

1. Antikrebswirkstoff umfassend zumindest ein Polypeptid ausgewählt aus der Gruppe bestehend aus den Polypeptiden (A1), (B1), (A2), (B2) und (C1) :
(A1): Polypeptid enthaltend eine Aminosäuresequenz umfassend die 1. bis 185. Region in der Aminosäuresequenz wie in der SEQ ID Nr. 1 gezeigt;
(B1): Polypeptid enthaltend eine Aminosäuresequenz umfassend die 232. bis 252. oder die 237. bis 252. Region in der Aminosäuresequenz wie in der SEQ ID Nr. 1 gezeigt;
(A2): Polypeptid enthaltend eine Aminosäuresequenz, in der die Aminosäurereste 1 bis 4 gelöscht, ersetzt oder der Aminosäuresequenz der 1. bis 185. Region in der Aminosäuresequenz wie in der SEQ ID Nr. 1 gezeigt zugefügt sind, und das eine Funktion zur Bindung an das menschliche ErbB2, gleichbedeutend mit der PTB Domäne des menschlichen FRS2β, besitzt;
(B2): Polypeptid enthaltend eine Aminosäuresequenz, in der die Aminosäurereste 1 bis 4 gelöscht, ersetzt oder der Aminosäuresequenz der 232. bis 252. oder der 237. bis 252. Region in der Aminosäuresequenz wie in der SEQ ID Nr. 1 gezeigt zugefügt sind, und das eine Funktion zur Bindung an das menschliche ERK2 besitzt; und
(C1): Polypeptid bestehend aus der Aminosäuresequenz wie in der SEQ ID Nr. 1 gezeigt;
zur Verwendung in einem Verfahren zur Krebsbehandlung durch Verabreichung des Antikrebswirkstoffs an Menschen, die hiervon betroffen sind, wobei der Krebs durch eine Überexpression des ErbB2 gekennzeichnet ist oder es sich um einen solchen Krebs handelt, bei dem der durch ErbB2 vermittelte Signalweg involviert ist.

2. Antikrebswirkstoff zur Verwendung gemäß Anspruch 1, umfassend zumindest ein Polypeptid ausgewählt aus der Gruppe bestehend aus den Polypeptiden (A1), (B1), (A2) und (B2):
(A1): Polypeptid bestehend aus einer Aminosäuresequenz umfassend die 1. bis 185. Region in der Aminosäuresequenz wie in der SEQ ID Nr. 1 gezeigt;
(B1): Polypeptid bestehend aus einer Aminosäuresequenz umfassend die 232. bis 252. oder die 237. bis 252. Region in der Aminosäuresequenz wie in der SEQ ID Nr. 1 gezeigt;
(A2): Polypeptid bestehend aus einer Aminosäuresequenz, in der die Aminosäurereste 1 bis 4 gelöscht, ersetzt oder der Aminosäuresequenz der 1. bis 185. Region in der Aminosäuresequenz wie in der SEQ ID Nr. 1 gezeigt zugefügt sind, und das eine Funktion zur Bindung an das menschliche ErbB2, gleichbedeutend mit der PTB Domäne des menschlichen FRS2β, besitzt; und
(B2): Polypeptid bestehend aus einer Aminosäuresequenz, in welcher die Aminosäurereste 1 bis 4 gelöscht, ersetzt oder der Aminosäuresequenz der 232. bis 252. oder 237. bis 252. Region in der Aminosäuresequenz wie in der SEQ ID Nr. 1 gezeigt zugefügt sind, und das eine Funktion zur Bindung an das menschliche ERK2 besitzt.

3. Antikrebswirkstoff umfassend zumindest eine Nukleinsäure, die ein entsprechendes Polypeptid ausgewählt aus der Gruppe bestehend aus den Polypeptiden (A1), (B1), (A2) und (B2), wie in Anspruch 1 oder 2 definiert, kodiert, zur Verwendung in einem Verfahren zur Krebsbehandlung durch Verabreichung des Antikrebswirkstoffs an Menschen, die hiervon betroffen sind, wobei der Krebs durch eine Überexpression des ErbB2 gekennzeichnet ist oder es sich um einen solchen Krebs handelt, bei dem der durch ErbB2 vermittelte Signalweg involviert ist.

4. Antikrebswirkstoff zur Verwendung gemäß Anspruch 3, umfassend zumindest eine Nukleinsäure ausgewählt aus der Gruppe bestehend aus den Nukleinsäuren (a1), (b1), (a2) und (b2) :
(a1): Nukleinsäure enthaltend eine Nukleotidsequenz umfassend die 1. bis 555. Region der Nukleotidsequenz wie in der SEQ ID Nr. 2 gezeigt;
(b1): Nukleinsäure enthaltend eine Nukleotidsequenz umfassend die 709. bis 756. Region der Nukleotidsequenz wie in der SEQ ID Nr. 2 gezeigt;
(a2): Nukleinsäure enthaltend eine Nukleotidsequenz, in der die Nukleotide 1 bis 6 gelöscht, ersetzt oder der Nukleotidsequenz der 1. bis 555. Region der Nukleotidsequenz wie in der SEQ ID Nr. 2 gezeigt zugefügt sind, und die für ein Polypeptid kodiert, das eine Funktion zur Bindung an das menschliche ErbB2, gleichbedeutend mit der PTB Domäne des menschlichen FRS2β, besitzt; und
(b2): Nukleinsäure enthaltend eine Nukleotidsequenz in der die Nukleotide 1 bis 6 gelöscht, ersetzt oder der Nukleotidsequenz der 709. bis 756. Region der Nukleotidsequenz wie in der SEQ ID Nr. 2 gezeigt zugefügt sind, und die für ein Polypeptid kodiert, das eine Funktion zur Bindung an das menschliche ERK2 besitzt.

5. Antikrebswirkstoff zur Verwendung gemäß Anspruch 3, umfassend zumindest eine Nukleinsäure ausgewählt aus der Gruppe bestehend aus den Nukleinsäuren (a1), (b1), (a2) und (b2) :
(a1): Nukleinsäure bestehend aus einer Nukleotidsequenz umfassend die 1. bis 555. Region der Nukleotidsequenz wie in der SEQ ID Nr. 2 gezeigt;
(b1): Nukleinsäure bestehend aus einer Nukleotidsequenz umfassend die 709. bis 756. Region der Nukleotidsequenz wie in der SEQ ID Nr. 2 gezeigt;
(a2): Nukleinsäure bestehend aus einer Nukleotidsequenz, in der die Nukleotide 1 bis 6 gelöscht, ersetzt oder der Nukleotidsequenz der 1. bis 555. Region der Nukleotidsequenz wie der in SEQ ID Nr. 2 gezeigt zugefügt sind, und die für ein Polypeptid kodiert, das eine Funktion zur Bindung an das menschliche ErbB2, gleichbedeutend mit der PTB Domäne des menschlichen FRS2β, besitzt; und
(b2): Nukleinsäure bestehend aus einer Nukleotidsequenz, in der die Nukleotide 1 bis 6 gelöscht, ersetzt oder der Nukleotidsequenz der 709. bis 756. Region der Nukleotidsequenz wie in der SEQ ID Nr. 2 gezeigt zugefügt sind, und die für ein Polypeptid kodiert, das eine Funktion zur Bindung an das menschliche ERK2 besitzt.

6. Antikrebswirkstoff zur Verwendung gemäß Anspruch 4, wobei die Nukleinsäure (a1) eine solche ist, die die Nukleotidsequenz der 1. bis zur 555. Region der Nukleotidsequenz, wie in der SEQ ID Nr. 2 gezeigt, umfasst, und die Nukleinsäure (b1) eine solche ist, die die Nukleotidsequenz der 709. bis 756. Region der Nukleotidsequenz, wie in der SEQ. ID Nr. 2 gezeigt, umfasst.

7. Antikrebswirkstoff zur Verwendung gemäß Anspruch 4, wobei die Nukleinsäuren (a1) oder (b1) dem menschlichen FRS2β-Gen entsprechen und die Nukleotidsequenz, wie in der SEQ ID Nr. 2 gezeigt, enthalten.

8. Antikrebswirkstoff zur Verwendung gemäß einem der vorhergehenden Ansprüche 3 bis 7, umfassend zumindest eine der Nukleinsäuren (a1) und (a2) und zumindest eine der Nukleinsäuren (b1) und (b2).

9. Antikrebswirkstoff zur Verwendung gemäß einem der Ansprüche 3 bis 8, umfassend eine chimäre Nukleinsäure umfassend zumindest eine der Nukleinsäuren (a1) und (a2) und zumindest eine der Nukleinsäuren (b1) und (b2).

10. Antikrebswirkstoff zur Verwendung gemäß einem der Ansprüche 3 bis 9, wobei die Nukleinsäure des Weiteren einen Vektor umfasst, und die Nukleinsäure mit diesem Vektor verknüpft ist.

11. Verwendung von zumindest einem Polypeptid ausgewählt aus der Gruppe bestehend aus den Polypeptiden (A1), (B1), (A2) und (B2), wie in Anspruch 1 definiert, oder zumindest einer Nukleinsäure, wie in einem der Ansprüche 2 bis 8 definiert, die ein entsprechendes Polypeptid kodieren, für die Herstellung eines Medikaments zur Krebsbehandlung, wobei der Krebs durch eine Überexpression des ErbB2 gekennzeichnet ist oder es sich um einen solchen Krebs handelt, bei dem der durch ErbB2 vermittelte Signalweg involviert ist.

12. Antikrebswirkstoff zur Verwendung nach einem der Ansprüche 1 bis 10 oder zur Verwendung gemäß Anspruch 11, wobei der Krebs ausgewählt ist aus der Gruppe bestehend aus Brustkrebs, Eierstockkrebs, Magenkrebs, Blasenkrebs, Mundkrebs, Speiseröhrenkrebs, Gehirntumor, Lungenkrebs, kraniozervikaler Krebs, Hautkrebs, Gebärmutterkrebs, Darmkrebs und Bauchspeicheldrüsenkrebs.

13. Substanz ausgewählt aus einem Antikörper, der spezifisch für das menschliche FRS2β ist, eine Sonde, die spezifisch für das menschliche FRS2β-Gen ist und ein Primer, der spezifisch für das menschliche FRS2β-Gen ist, zur Verwendung in einem Verfahren zur Diagnose der Notwendigkeit einer Behandlung einer menschlichen Krebszelle mit einem Antikrebswirkstoff, wobei für den Fall, dass eine Herunterregulierung der FRS2β-Signalwirkung nachgewiesen wird, eine Behandlung mit einem auf das ErbB2 zielenden Antikrebswirkstoffs notwendig ist.

14. In-vitro-Verfahren zur Bestimmung des Vorliegens einer Herunterregulierung eines durch ErbB2 vermittelten Signalwegs in einer menschlichen Zelle, wobei das Verfahren einen Schritt des Nachweisens eines Markers für die Herunterregulierung, der aus dem menschlichen FRS2β und dem Transkriptionsprodukt des FRS2β-Gens zumindest eines enthält, umfasst, wobei der Nachweis von FRS2β darauf hindeutet, dass der ErbB2-Signalweg herunterreguliert ist.

15. In-vitro-Diagnoseverfahren zur Bestimmung der Notwendigkeit einer Krebsbehandlung mit einem Antikrebswirkstoff, wobei das Verfahren einen Schritt des Nachweisens eines Markers, der aus dem menschlichen FRS2β und dem Transkriptionsprodukt des FRS2β-Gens zumindest eines enthält, umfasst, wobei für den Fall, dass eine Herunterregulierung der FRS2β-Signalwirkung nachgewiesen wird, eine Behandlung mit einem auf das ErbB2 zielenden Antikrebswirkstoffs notwendig ist.

16. Verfahren gemäß Anspruch 14 oder 15, wobei für den Nachweis des Markers ein Antikörper verwendet wird, der spezifisch für das menschliches FRS2β ist, eine Sonde, die spezifisch für das menschliche FRS2β-Gen ist und ein Primer, der spezifisch für das menschliche FRS2β-Gen ist.

## Revendications

1. Médicament anticancéreux comprenant au moins un polypeptide sélectionné dans le groupe constitué des polypeptides (A1), (B1), (A2), (B2) et (C1) :
(A1) : un polypeptide comprenant une séquence d'acides aminés comprenant la 1^{e} à la 185^{e} région de la séquence d'acides aminés représentée en SEQ ID NO: 1 ;
(B1) : un polypeptide comprenant une séquence d'acides aminés comprenant la 232^{e} à la 252^{e} ou la 237^{e} à la 252^{e} région de la séquence d'acides aminés représentée en SEQ ID NO: 1 ;
(A2) : un polypeptide comprenant une séquence d'acides aminés dans laquelle 1-4 résidus d'acides aminés sont supprimés, remplacés ou ajoutés dans la séquence d'acides aminés de la 1^{e} à la 185^{e} région de la séquence d'acides aminés représentée en SEQ ID NO: 1, et ayant une fonction de liaison à l'ErbB2 humaine équivalente au domaine PTB de la FRS2β humaine ;
(B2) : un polypeptide comprenant une séquence d'acides aminés dans laquelle 1-4 résidus d'acides aminés sont supprimés, remplacés ou ajoutés dans la séquence d'acides aminés de la 232^{e} - la 252^{e} ou la 237^{e} - la 252^{e} région dans la séquence d'acides aminés montrée dans SEQ ID NO: 1, et ayant une fonction de liaison à l'ERK2 humaine ; et
(C1) un polypeptide consistant en la séquence d'acides aminés montrée dans SEQ ID NO: 1 ;
pour son utilisation dans une méthode de traitement du cancer par administration dudit médicament anticancéreux à un individu en ayant besoin, étant entendu que le cancer est **caractérisé par** une surexpression d'ErbB2 ou est un cancer dans lequel la voie de signalisation médiée par ErbB2 est impliquée.

2. Médicament anticancéreux pour son utilisation selon la revendication 1, comprenant au moins un polypeptide sélectionné dans le groupe constitué des polypeptides (A1), (B1), (A2) et (B2) :
(A1) : un polypeptide consistant en une séquence d'acides aminés comprenant la 1^{e} à la 185^{e} région de la séquence d'acides aminés représentée en SEQ ID NO: 1 ;
(B1) : un polypeptide consistant en une séquence d'acides aminés comprenant la 232^{e} à la 252^{e} ou la 237^{e} à la 252^{e} région de la séquence d'acides aminés représentée en SEQ ID NO: 1 ;
(A2) : un polypeptide consistant en une séquence d'acides aminés dans laquelle 1-4 résidus d'acides aminés sont supprimés, remplacés ou ajoutés dans la séquence d'acides aminés de la 1^{e} à la 185^{e} région de la séquence d'acides aminés représentée en SEQ ID NO: 1, et ayant une fonction de liaison à l'ErbB2 humaine équivalente au domaine PTB de la FRS2β humaine ; et
(B2) : un polypeptide consistant en une séquence d'acides aminés dans laquelle 1-4 résidus d'acides aminés sont supprimés, remplacés ou ajoutés dans la séquence d'acides aminés de la 232^{e} à la 252^{e} ou la 237^{e} à la 252^{e} région dans la séquence d'acides aminés représentée en SEQ ID NO: 1, et ayant une fonction de liaison à l'ERK2 humaine.

3. Médicament anticancéreux comprenant au moins un acide nucléique codant pour un polypeptide respectif sélectionné dans le groupe constitué des polypeptides (A1), (B1), (A2) et (B2) tels que définis dans la revendication 1 ou 2, pour son utilisation dans une méthode de traitement du cancer par administration dudit médicament anticancéreux à un individu en ayant besoin, étant entendu que le cancer est **caractérisé par** une surexpression d'ErbB2 ou est un cancer dans lequel la voie de signalisation médiée par ErbB2 est impliquée.

4. Médicament anticancéreux pour son utilisation selon la revendication 3, comprenant au moins un acide nucléique sélectionné dans le groupe constitué des acides nucléiques (a1), (b1), (a2) et (b2) :
(a1) : un acide nucléique comprenant une séquence de nucléotides comprenant la le à la 555^{e} région de la séquence de nucléotides représentée en SEQ ID NO: 2 ;
(b1) : un acide nucléique comprenant une séquence de nucléotides comprenant la 709^{e} à la 756^{e} région de la séquence de nucléotides représentée en SEQ ID NO: 2 ;
(a2) : un acide nucléique comprenant une séquence de nucléotides dans laquelle 1-6 nucléotides sont supprimés, remplacés ou ajoutés dans la séquence de nucléotides de la 1^{e} à la 555^{e} région de la séquence de nucléotides représentée en SEQ ID NO: 2, et codant pour un polypeptide ayant une fonction de liaison à l'ErbB2 humaine équivalente au domaine PTB de la FRS2β humaine ; et
(b2) : un acide nucléique comprenant une séquence de nucléotides dans laquelle 1-6 nucléotides sont supprimés, remplacés ou ajoutés dans la séquence de nucléotides de la 709^{e} à la 756^{e} région de la séquence de nucléotides représentée en SEQ ID NO: 2, et codant pour un polypeptide ayant une fonction de liaison à l'ERK2 humaine.

5. Médicament anticancéreux pour son utilisation selon la revendication 3, comprenant au moins un acide nucléique sélectionné dans le groupe constitué des acides nucléiques (a1), (b1), (a2) et (b2) :
(a1): un acide nucléique consistant en une séquence de nucléotides comprenant la 1^{e} à la 555^{e} région de la séquence de nucléotides représentée en SEQ ID NO: 2 ;
(b1) : un acide nucléique consistant en une séquence de nucléotides comprenant la 709^{e} à la 756^{e} région de la séquence de nucléotides représentée en SEQ ID NO: 2 ;
(a2) : un acide nucléique consistant en une séquence de nucléotides dans laquelle 1-6 nucléotides sont supprimés, remplacés ou ajoutés dans la séquence de nucléotides de la 1^{e} à la 555^{e} région de la séquence de nucléotides représentée en SEQ ID NO: 2, et codant pour un polypeptide ayant une fonction de liaison à l'ErbB2 humaine équivalente au domaine PTB de la FRS2β humaine ; et
(b2) : un acide nucléique consistant en une séquence de nucléotides dans laquelle 1-6 nucléotides sont supprimés, remplacés ou ajoutés dans la séquence de nucléotides de la 709^{e} à la 756^{e} région de la séquence de nucléotides représentée en SEQ ID NO: 2, et codant pour un polypeptide ayant une fonction de liaison à l'ERK2 humaine.

6. Médicament anticancéreux pour son utilisation selon la revendication 4, où ledit acide nucléique (a1) est un acide nucléique comprenant la séquence de nucléotides de la 1^{e} à la 555^{e} région de la séquence de nucléotides représentée en SEQ ID NO: 2, et ledit acide nucléique (b1) est un acide nucléique comprenant la séquence de nucléotides de la 709^{e} à la 756^{e} région de la séquence de nucléotides représentée en SEQ ID NO: 2.

7. Médicament anticancéreux pour son utilisation selon la revendication 4, où les acides nucléiques dudit (a1) ou (b1) sont le gène FRS2β humain comprenant la séquence de nucléotides représentée en SEQ ID NO: 2.

8. Médicament anticancéreux pour son utilisation selon l'une quelconque des revendications 3 à 7, comprenant au moins un des acides nucléiques de (a1) et (a2) et au moins un des acides nucléiques de (b1) et (b2).

9. Médicament anticancéreux pour son utilisation selon l'une quelconque des revendications 3 à 8, comprenant un acide nucléique chimérique comprenant au moins un des acides nucléiques de (a1) et (a2) et au moins un des acides nucléiques de (b1) et (b2).

10. Médicament anticancéreux pour son utilisation selon l'une quelconque des revendications 3 à 9, où ledit acide nucléique comprend en outre un vecteur et ledit acide nucléique est lié audit vecteur.

11. Utilisation d'au moins un polypeptide sélectionné dans le groupe constitué des polypeptides (A1), (B1), (A2) et (B2) tels que définis dans la revendication 1 ou d'au moins un acide nucléique tel que défini dans l'une quelconque des revendications 2 à 8 codant pour un desdits polypeptides respectifs, pour la préparation d'un médicament destiné au traitement du cancer, étant entendu que le cancer est **caractérisé par** une surexpression d'ErbB2 ou est un cancer dans lequel la voie de signalisation médiée par ErbB2 est impliquée.

12. Médicament anticancéreux pour son utilisation selon l'une quelconque des revendications 1 à 10 ou utilisation selon la revendication 11, où le cancer est sélectionné dans le groupe constitué du cancer du sein, du cancer de l'ovaire, du cancer de l'estomac, du cancer de la vessie, du cancer de la bouche, du cancer de l'oesophage, d'une tumeur cérébrale, du cancer du poumon, du cancer craniocervical, du cancer cutané, du cancer de l'utérus, du cancer du côlon et du cancer du pancréas.

13. Substance sélectionnée parmi un anticorps spécifique pour la FRS2β humaine, une sonde spécifique pour le gène FRS2β humain et une amorce spécifique pour le gène FRS2β humain, pour son utilisation dans une méthode de diagnostic de la nécessité d'un traitement d'une cellule cancéreuse humaine avec un médicament anticancéreux, où lorsqu'une régulation négative de la signalisation de FRS2β est détectée, un traitement avec un médicament anticancéreux ciblé vers ErbB2 est nécessaire.

14. Méthode *in vitro* pour déterminer la présence d'une régulation négative d'une voie de signalisation médiée par ErbB2 dans une cellule humaine, la méthode comprenant une étape de détection d'un marqueur de régulation négative comprenant au moins l'un d'une FRS2β humaine et d'un produit de transcription du gène FRS2β, où la détection de FRS2β indique que la voie de signalisation ErbB2 est négativement régulée.

15. Méthode de diagnostic *in vitro* pour déterminer la nécessité d'un traitement du cancer avec un médicament anticancéreux, la méthode comprenant une étape de détection d'un marqueur comprenant au moins l'un d'une FRS2β humaine et d'un produit de transcription du gène FRS2β, où lorsqu'une régulation négative de la signalisation de FRS2β est détectée, un traitement avec un médicament anticancéreux ciblé vers ErbB2 est nécessaire.

16. Méthode selon la revendication 14 ou la revendication 15, dans laquelle le marqueur est détecté en utilisant un anticorps spécifique pour la FRS2β humaine, une sonde spécifique pour le gène FRS2β humain et une amorce spécifique pour le gène FRS2β humain.
